# FASCICULE DE BREVET EUROPEEN

(11) **EP 1 021 457 B1**
(45) Date de publication et mention de la délivrance du brevet: **09.04.2003**
(21) Numéro de dépôt: 97931877.1
(22) Date de dépôt: 04.07.1997
(51) Int. Cl.: C07H 19/048, C07H 15/26, C07H 17/02, C07D 213/14, C07D 213/69, A61K 31/70, A61K 31/44

(54) **PROCEDE DE SYNTHESE REGIOSPECIFIQUE DE NOUVEAUX DERIVES 3-HYDROXYPYRIDIN-4(1H)-0NES A PARTIR D'AMINOITOLS, PRODUITS OBTENUS PAR CE PROCEDE ET LEURS APPLICATIONS**
VERFAHREN ZUR REGIOSELEKTIVEN SYNTHESE VON 3-HYDROXY-PYRIDIN-4-(1H)-ONE-DERIVATEN AUS AMINOITOLEN, NACH DIESEM VERFAHREN HERGESTELLTE PRODUKTE UND DEREN VERWENDUNG
METHOD FOR THE SITE SPECIFIC SYNTHESIS OF NOVEL 3-HYDROXYPYRIDIN-4(1H)-ONES DERIVATIVES FROM AMINOITOLS, PRODUCTS OBTAINED BY THIS METHOD AND THEIR APPLICATIONS

(30) Priorité: 05.07.1996 FR 9608382
(43) Date de publication de la demande: 26.07.2000
(73) Titulaire: Istituto Biochimico Pavese Pharma SPA, 27100 Pavia (IT)
(72) Inventeur: VANLEMMENS, Pierre, Paul, F-80000 Amiens (FR); POSTEL, Denis, Ghislain, F-80000 Amiens (FR); GERMAIN, Pierig, Emmanuel, F-80580 Pont Remy (FR); JULIEN, René, Jean-Marie, F-92160 Antony (FR); PETIT, Jean-Pierre, Constant, F-80000 Amiens (FR); RONCO, Gino, Lino, F-80000 Amiens (FR); VILLA, Pierre, Joseph, F-80000 Amiens (FR)
(74) Mandataire: Gervasi, Gemma, Dr.
(86) Numéro de dépôt international: FR9701211
(87) Numéro de publication internationale: WO98001458

(56) Documents cités:
- US-A- 4 209 613
- G. LIU ET AL: "Synthesis of 2-alkyl-3-hydroxy-4-pyridinone-ribonucleos ides, potential oral iron chelators." NUCLEOSIDES & NUCLEOTIDES, vol. 14, no. 9&10, 1995, pages 1901-1904, XP000647903
- D. T. MAO ET AL: "Synthesis of 3-hydroxy-2 and 4-pyridone nucleosides as potential antitumor agents." JOURNAL OF MEDICINAL CHEMISTRY, vol. 27, no. 2, 1984, WASHINGTON US, pages 160-164, XP000647940

## Description

La présente invention a trait à un procédé de préparation régiospécifique de nouveaux dérivés 3-hydroxypyridin-4(*1H*)-ones à partir d'itols

Elle concerne également les produits obtenus par ce procédé ainsi que leurs applications, notamment comme agent complexant de Fe^{III}

On sait que les composés de type 3-hydroxypyridin-4(*1H*)-ones complexent le fer a l'état d'oxydation III. Ils sont utilisés, notamment dans les traitements chez l'Homme visant à limiter les surcharges toxiques en Fe^{III} (Kontoghiorghes G J et coll., *Lancet*, 1294-1295 (1987)) Ces surcharges peuvent être d'origine endogène comme par exemple celles rencontrées chez les malades atteints de sidérose ou encore exogène comme dans le cas des intoxications induites par les polytransfusions massives et fréquentes auxquelles sont soumis les malades atteints notamment de β-Thalassémie

Il est également connu que la toxicité de ces composés, pour l'homme, est moindre quand le site -2 du cycle pyridinique est substitué par un groupement alkyle et ce, sans modifier son pouvoir chélateur vis à vis de Fe^{III} (Hershko C et coll , *Ann. N Y. Acad. Sci.*, 612, 351-360, (1990)).

Marquez et coll. ont préparé des dérivés glycosidiques de la 2-méthyl-3-hydroxypyridin-4(*1H*)-ones par condensation du maltol benzylé sur le D-ribose et le D-arabinose, activés en anomérique sous forme d'acétate et dont les autres groupements hydroxylés sont protégés sous forme de benzoate ou d'acétate (Marquez et coll, *J. Med. Chem*., 27 (2), 160-164 (1984)).

Miller et coll. en utilisant la même stratégie ont préparé deux ribosides de 2-méthyl- et 2-éthyl-3-hydroxypyridin-4(*1H*)-one de configuration β, par mise en réaction de la 1-triméthylsilyl-2-alkyl-3-benzylpyridin-4(*1H*)-one sur les 1,2,3,4-tétra-*O*-acétyl-β-D-ribopyranose et le 1-*O*-acétyl-2,3,5-tri-*O*-benzoyl-β-D-ribofuranose en présence de SnCl₄ (Liu G. et coll., *Nucleosides, Nucleotides,* 14 (9-10), 1901-1904 (1995)).

Un étude sur la perméabilité intestinale de certaines substances parmi lesquelles des saccharides a été publié par Travis S. et Menies I. (Chemical Science, 1992,82, 471-488).

L'un des buts de la présente invention est de décrire un procédé de synthèse régiospécifique de nouveaux dérivés de 3-hydroxypyridin-4(*1H*)-ones à partir d'aminoitols de formule générale: 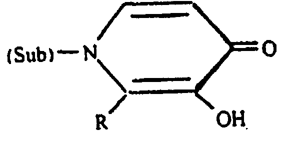 dans laquelle R représente un radical alkyle ou alkylène ou haloalkyle ou haloalkylène, les halogènes étant choisis de préférence parmi chlorure et fluorure, droit ou ramifié, ayant de 1 à 4 atomes de carbone et ayant des hétéroatomes ou non, et Sub représente un itol, cyclique ou non, protégé ou non, choisi par exemple parmi hexitol, pentitol, tétritol ou glycérol et de préférence parmi glucitol, galacitol, mannitol, xylitol, érythritol, glycérol, dont le squelette hydrocarboné est lié à l'atome d'azote de la pyridinone soit directement, soit par l'intermédiaire d'un bras d'espacement de type alkylène ou haloalkylène, l'halogène étant choisi de préférence parmi chlorure et fluorure, droit ou ramifié, ayant de 1 à 4 atomes de carbone, des hétéroatomes ou non.

La présente invention est caractérisée en ce que le procédé comprend :
- une première étape. (étape a) de protection du groupement 3-hydroxy du dérivé pyranone ;
- une deuxième étape (étape b) de substitution de l'atome d'oxygène intracyclique de la pyranone par l'atome d'azote de la fonction amine de l'aminoitol, pour conduire à la 2-R-3-hydroxypyridin-4(*1H*)-one dérivée de l'itol ;
- une troisième étape (étape c) de déprotection du groupement 3-OH du cycle pyridinone et éventuellement des groupements OH de l'itol.
   Les groupements OH de l'itol peuvent éventuellement également ètre déproteges dans une quatrième étape (étape d) 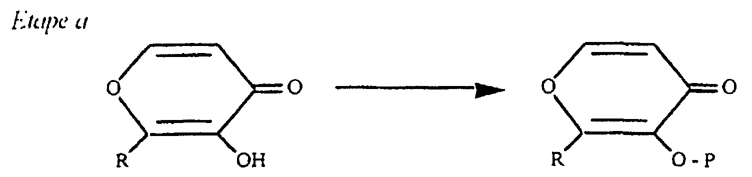
   Dans l'étape (a) de protection du groupement 3-hydroxy du dérivé pyranone, on fait réagir le dérivé 2-R-3-hydroxypyran-4-one avec au moins un équivalent molaire de derivé P-X, dans lequel P représente un groupement alkyle, cyclique ou non. ramifié ou non. saturé ou non ou encore un groupement phényle substitué ou non ou encore un groupement aryle substitué ou non, et de préférence choisi parmi allyle, benzyle, phényle et X est un groupement partant choisi par exemple parmi halogénure et sulfonate et de préference choisi parmi chlorure, bromure, iodure, tosylate, mésylate, brosylate. nosylate. tritlate. L'étape (a) de protection est conduite en présence d'au moins 1 équivalent molaire de base forte choisie par exemple parmi les bases hydroxylées ou encore parmi les alcanoates et les sels d'acides faibles, le cation pouvant être monovalent tel que Na⁺, K⁺ Li⁺, Rb⁺, Cs⁺ ou encore un cation polyvalent Mⁿ⁺ de type alcalino-terreux ou tout autre On peut utiliser comme solvant de réaction des mélanges hydroalcanoliques, l'alcanol etant choisi par exemple parmi le méthanol, l'éthanol, le propanol ou l'isobutanol ou encore un alcanol seul, ou encore un solvant aprotique polaire choisi par exemple parmi l'hexaméthylphosphorotriamide (HMPA), le diméthylformamide (DMF), le diméthoxyéthane (DME), le diméthylsulfoxyde (DMSO), l'acétone , ou encore un mélange de ces derniers, le solvant aprotique polaire pouvant être ou non associé à un solvant aprotique apolaire choisi par exemple parmi les solvants aromatiques, les hydrocarbures ou encore les éthéroxydes ou encore un mélange de ces solvants
   De plus, l'étape (a) comprend les opérations suivantes .
- élimination du solvant et reprise du résidu par un solvant organique.
- lavage de la phase organique successivement par une solution aqueuse de base. comme précedemment définie, puis par de l'eau ,
- évaporation des phases organiques précédemment obtenues, pour conduire au dérive pyranone protégé en -3

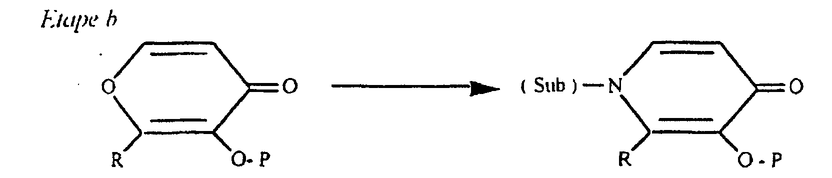

Dans l'étape (b) de substitution de l'atome d'oxygène intracyclique de la pyranone par l'atome d'azote de l'aminoitol, on fait réagir au moins 1 équivalent molaire de la pyranone protégée issue de l'étape (a), en présence d'une base forte comme définie à l'étape (a), sur 1 équivalent molaire de l'aminoitol, portant le groupement amino soit directement sur le reste saccharidique soit par l'intermédiaire d'un bras d'espacement

Les aminoitols sont obtenus dans les conditions classiques de la littérature, consistant en la condensation directe d'ammoniac sur un monosaccharide ou un itol activé (Fletchner T W., *Carbohydr. Res.* , 77, 262 (1979)), ou encore consistant en la préparation de dérivés azotures de itol, suivie de la réduction en derives amino par une hydrogénation catalytique ou par un hydrure métallique (Scriven E F V *Chem. Reviews,* 88, 2, (1988)) ou encore PPh₃-H₂O (Mungall W S et coll *J. Org. Chem.,* 40, 11 (1975)).

On peut utiliser comme solvant de l'étape (b) des mélanges hydroalcanoliques. l'alcanol étant choisi par exemple parmi le méthanol, l'éthanol, le propanol ou l'isobutanol ou encore un alcanol seul, ou encore un solvant aprotique polaire choisi par exemple parmi l'hexaméthylphosphorotriamide (HMPA), le diméthylformamide (DMF), le dimethoxyéthane (DME), le diméthylsulfoxyde (DMSO), l'acétone , ou encore un mélange de ces derniers. le solvant aprotique polaire pouvant être ou non associé à un solvant aprotique apolaire choisi par exemple parmi les solvants aromatiques, les hydrocarbures ou encore les éthéroxydes ou encore un mélange de ces solvants De plus l'étape (b) comprend les operations suivantes
- neutralisation de la base par addition d'un acide minéral ou organique.
- elimination du solvant organique,
- reprise du résidu par un solvant organique et lavage de la phase organique par de l'eau,
- evaporation de la phase organique,
- purification du résidu par recristallisation ou chromatographie

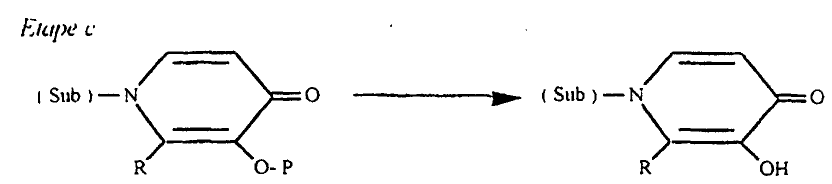

L'étape (c) consiste en la déprotection du cycle pyridinone des composés issus de l'étape b Selon la nature des groupements protecteurs P introduits lors de l'étape (a), la deprotection peut être réalisée par hydrogénolyse par exemple pour P choisi par exemple parmi benzyle, phényle, allyle ; ou encore par exemple lorsque P est un groupement allyle, par hydrolyse acidocatalysée, après isomérisation basocatalysée du groupement allyle en groupement propényle

On peut choisir comme solvant de réaction pour la déprotection par hydrogénolyse, un alcanol ou encore un mélange alcanol-eau, l'alcanol étant choisi par exemple parmi le méthanol, l'éthanol, le propanol, l'isopropanol , ou encore un solvant organique associé ou non à l'eau et permettant la solubilisation du produit déprotege. choisi par exemple parmi tétrahydrofurane, dioxane

On peut choisir comme catalyseur d'hydrogénolyse le Pd/C, ou encore tout autre catalyseur d'hydrogénolyse choisi par exemple parmi les dérivés du platine, les dérivés du nickel.

De plus l'étape (c) comprend les opérations suivantes
- filtration du catalyseur ,
- élimination du solvant organique,
- purification du résidu par exemple par recristallisation dans par exemple un mélange binaire de solvants organiques choisis par exemple parmi hexane, acétone

L'étape (c) peut avantageusement ètre conduite. dans le cas de composes a reste de l'itol protégé par des groupements acidolabiles, dans les conditions décrites precedemment, en présence d'un catalyseur acide de façon à opérer la déprotection des groupements protecteurs du reste de l'itol de façon "one-pot" Le dérivé de l'itol de la 2-R-3-hydroxypyridin-4(*1H*)-one peut alors être obtenu soit sous forme de sel d'ammonium par exemple par lyophilisation du milieu réactionnel apres élimination du solvant organique et addition d'eau, soit sous forme d'amine après neutralisation du milieu réactionnel par exemple par passage sur résine échangeuse d'ion de nature basique choisie par exemple parmi Dowex® Amberlyst® ou Amberlite®

Les composés issus de l'étape (c) dont le reste de l'itol possède des groupements hydroxyles protégés peuvent être soumis à une étape (d) de déprotection des groupements hydroxyles selon les conditions classiques de la littérature choisies en fonction de la nature de ces mêmes groupements protecteurs

Un autre but de la présente invention est de fournir de nouvelles 2-R-3-hydroxypyridin-4(*1H*)-ones , dérivés d'itols, répondant à la formule suivante 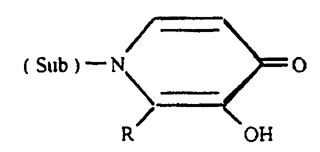 comme défini précédemment qui de par leurs propriétés chimiques peuvent trouver une application comme chélatant des métaux et plus particulièrement comme médicament pour le traitement des surcharges toxiques en Fe^{III}

Elle a également pour objet des médicaments pour le traitement des surcharges toxiques en métaux comportant une quantité thérapeutiquement efficace de l'un au moins des composés selon l'invention dans un véhicule administrable à l'Homme. Ce vehicule peut être un vehicule usuel pour une administration orale ou intramusculaire. Le médicament est administré en une ou plusieurs fois. De préférence le médicament est préparé sous forme de doses comprenant entre 30 et 150 mg/kg/jour de poids corporel L'invention a également pour objet l'utilisation des nouveaux composes selon l'invention pour la préparation d'un médicament destiné au traitement des surcharges toxiques en métaux.

Le traitement est notamment utile pour les surcharges toxiques en Fe^{III} Parmi ces surcharges toxiques figurent notamment les hémoglobinopathies, la β-thalassémie, la drepanocytose .

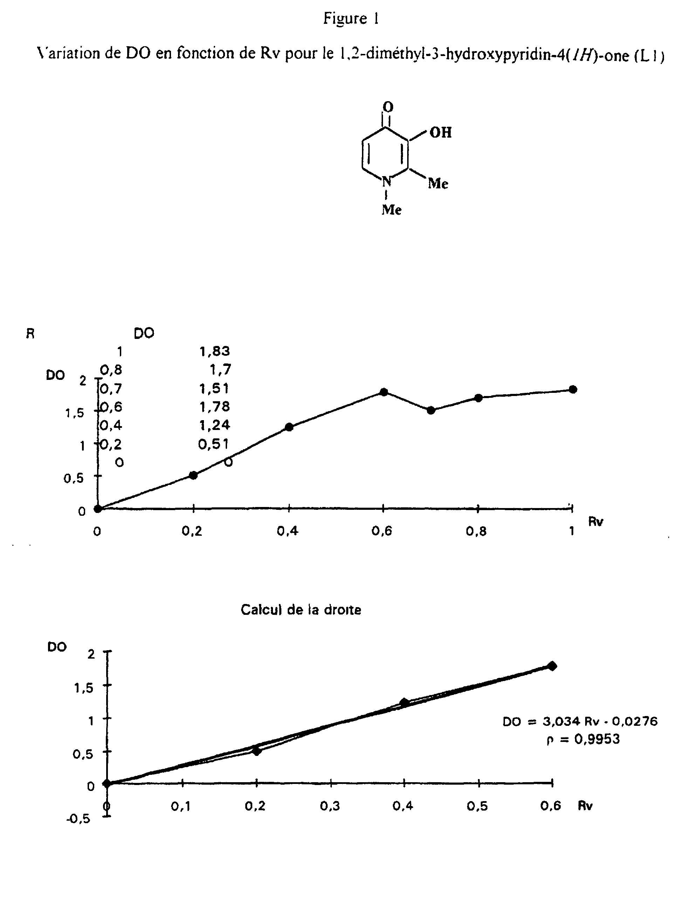

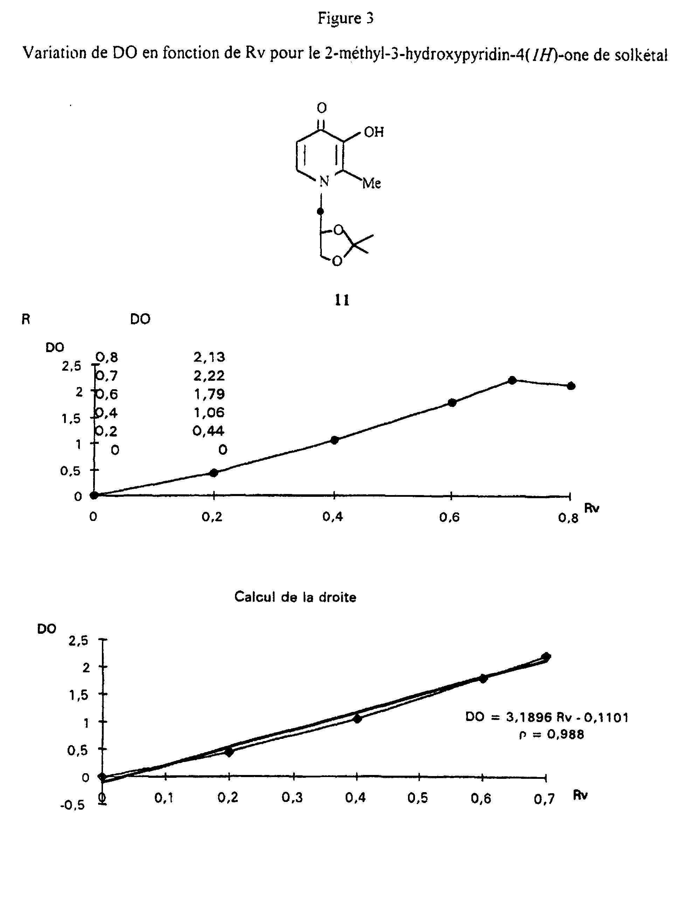

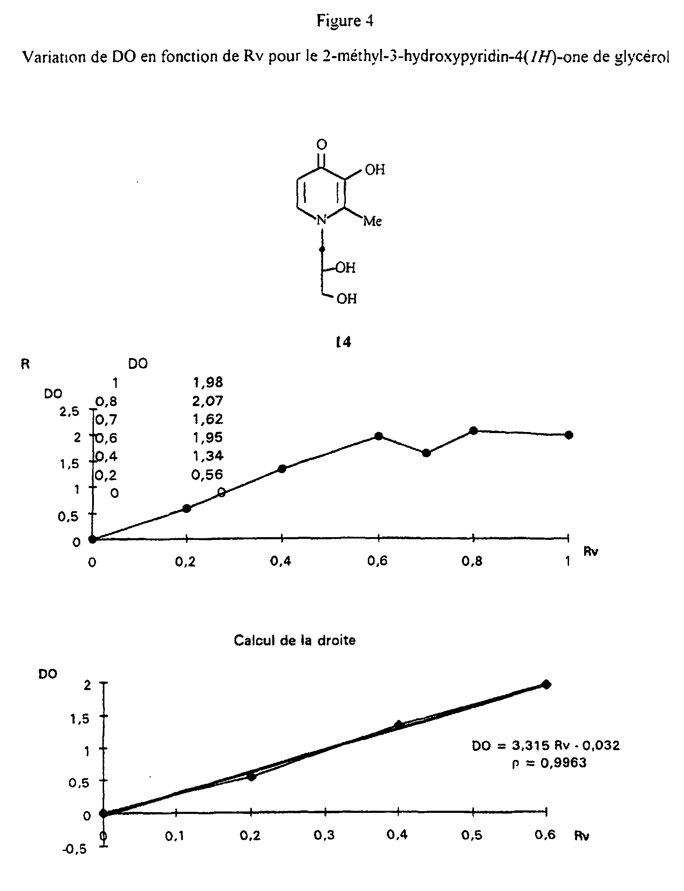

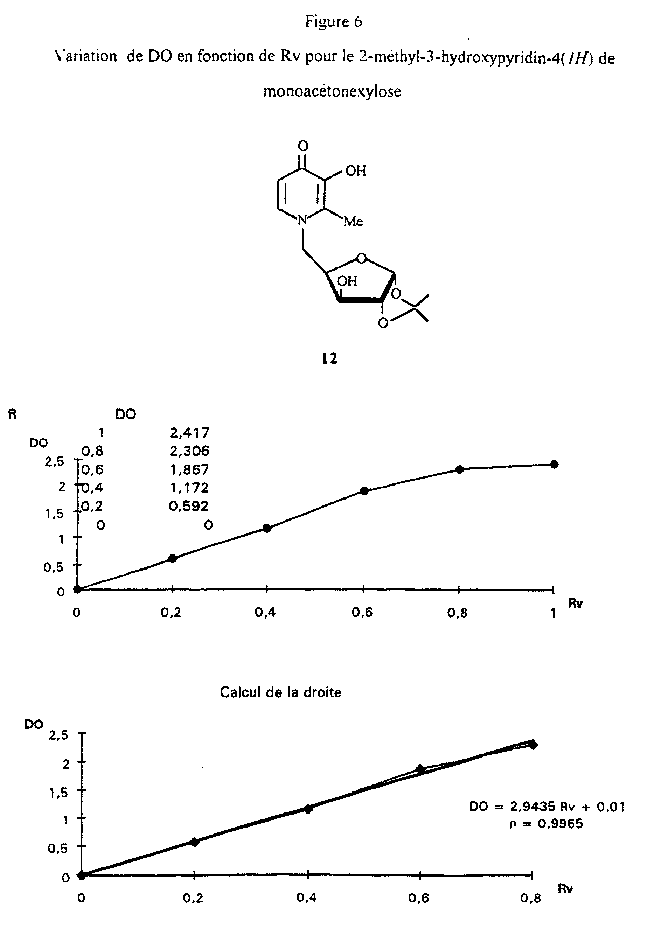

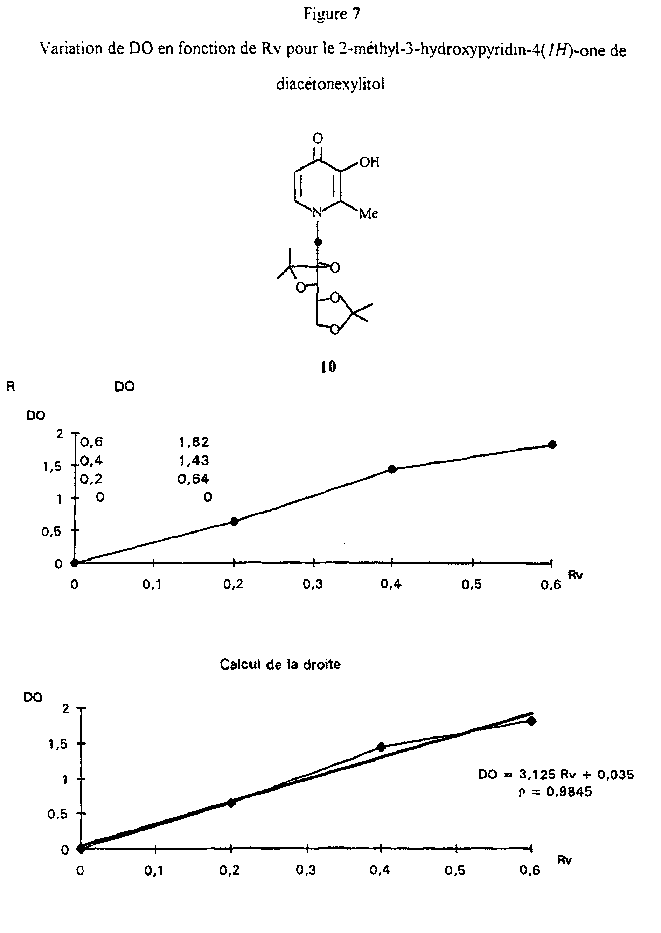

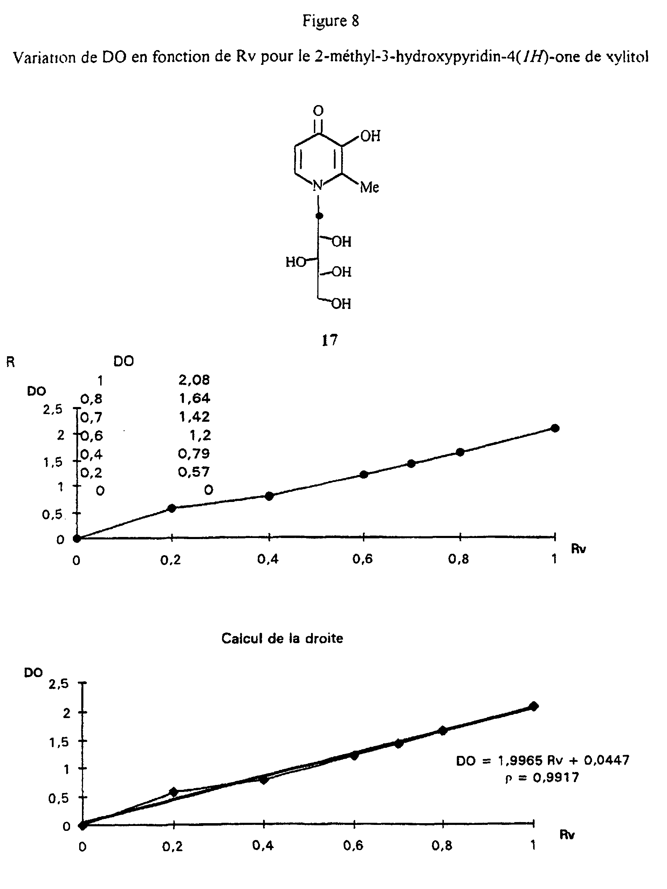

Les figures 1 à 9 représentent, pour différents composés, la variation de densite optique (DO) en fonction du rapport Rv (Rv= Volume de solution de Ligand/ Volume de solution de Fe^{III})

### Exemple 1 Synthèse du 2-méthvl-3-benzyloxy-4-pyranone (1)

20,0 g (159 mmol) de 2-méthyl-3-hydroxy-4-pyranone, 22,1 g (175 mmol, 1,1 eq) de chlorure de benzyle sont introduits dans 200 mL de méthanol, on ajoute ensuite 6.98 g (175 mmol) de NaOH dans 22 mL d'eau Le milieu réactionnel est porté sous agitation au reflux pendant 6h30 Ensuite le solvant est évaporé et le résidu repris par 100 mL de dichlorométhane Cette solution est lavée successivement par une solution aqueuse de soude a 5% (2x80 mL) et par de l'eau (2x80 mL). Les phases organiques sont rassemblées sechees sur sulfate de sodium, filtrées et évaporées sous pression réduite On isole 32.3 g (149 mmol) de 2-méthyl-3-benzyloxy-4-pyranone. Rendement : 94% 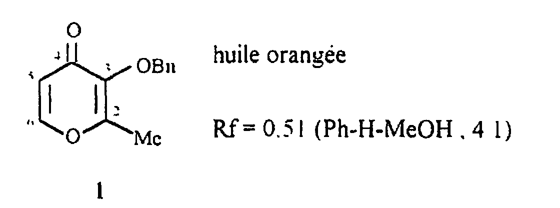 RMN ¹H δ 8,00 (d, J_{5,6} = 5,7 Hz, H-6), 7,42-7,30 (m, 5H Ph), 6,37 (d, J_{5,6} = 5,7 Hz, H-5), 5,04 (s,CH₂-Ph), 2,10 (s, CH₃),
RMN ¹H dans CDCl₃ : δ 7,55 (d, H-6), 7.40 (s, 5H, H aromatiques), 6,35 (d, H-5). 5.04 (s. CH₂-Ph), 2,10 (s, CH₃);
RMN ¹³C δ 173,9 (C-4), 159,1 (C-3), 154,8 (C-6), 143.1 (C-2), 136,6 (C_{Ipso}). 128,5 (2x Cₒᵣₜₕₒ), 128,2 (2x Cₘₑₜₐ), 128,0 (Cₚₐᵣₐ), 116,4 (C-5), 72,6 (CH₂-Ph), 14,3 (CH₃).

### Exemple 2 Synthèse du 1-(2',3' 4',5'-di-O-isopropylidène-xylityl)-2-méthyl-3-benzyloxypyridin-4(1H)-one (noté 2-méthyl-3-benzyloxypyridin-4(1H)-one de diacétonexylitol) (2)

20,0 g (92,5 mmol, 1,5 éq) de 2-méthyl-3-benzyloxy-4-pyranone. 14,3 g (61.8 mmol) de 1-amino-1-désoxy-2,3 4,5-di-*O*-isopropylidène-xylitol sont introduits dans 350 mL d'un mélange éthanol-eau 50-50 puis on ajoute 8 mL d'une solution aqueuse de soude (2N). Le milieu réactionnel est alors porté sous agitation au reflux pendant 3 jours On laisse refroidir le milieu réactionnel et on le neutralise en ajoutant une solution de HCl concentré Après évaporation de l'éthanol, on extrait le résidu par un mélange diçhlorométhane-eau 60 40 v/v (400 mL) Les phases organiques sont rassemblées, séchees sur Na₂SO₄ anhydre. filtrées puis évaporées sous pression réduite. Le résidu est recristallise dans un mélange hexane-acétone On isole 10.2 g (23,7 mmol) de 2-méthyl-3-benzyloxypyridin-4(1H)-one de diacétonexylitol, pur d'après les analyses chromatographiques et RMN. Rendement 38% 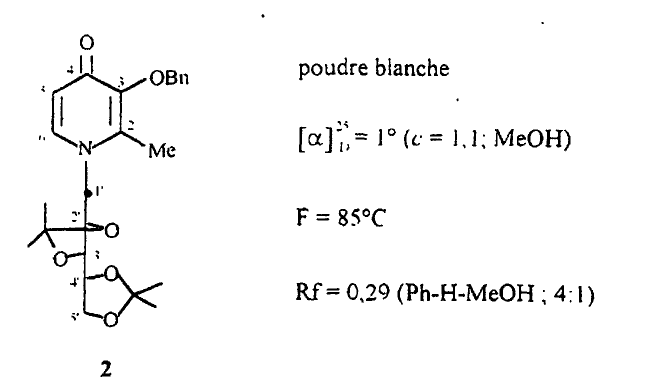 RMN ¹H sucre : δ 4,23-4,05 (massif, H-1'_{a,} H-1'_{b,} H-2', H-4', H-5'ₐ), 3.84 (dd, J_{3',4'} = 7.6, Hz. J_{2' 3'}= 7. H-3'), 3,72 (dd, H-5'_{b}), 1,33 (s, CH₃), 1,31 (s, CH₃), 1,29 (s, CH₃), 1.29 (s, CH₃).
hétérocycle : δ 7,54 (d, J_{5,6} = 7,6 Hz, H-6), 7,42 - 7,31 (m, 5H Ph), 6,21 (d, J_{5,6} = 7,6 Hz, H-5), 5,02 (2 dd, Hₐ CH₂-Ph), H_{b} CH₂-Ph), 2,20 (s, CH₃),
RMN ¹³C sucre : δ 109,4 (C_{Ip}), 108,7 (C_{Ip}), 77,3 (C-3'), 76,0 (C-2'), 73,8 (C-4'). 64,8 (C-5'). 54,1 (C-1'), 26,7 (CH₃), 26,5 (CH₃), 25,9 (CH₃), 25,3 (CH₃),
hétérocycle : δ 171,9 (C-4), 144,9 (C-2), 141,0 (C-3), 140,0 (C-6), 137.6 (C_{Ipso}), 128,4 (2xCₒᵣₜₕₒ), 128,1 (2xC_{méta}), 127,7 (Cₚₐᵣₐ), 115,7 (C-5), 71,7 (CH₂-Ph), 13,3 (CH₃)

### Exemple 3 Synthèse du 1-(2',4' 3',5'-di-O-méthylène-xylityl)-2-méthyl-3-benzyloxypyridin-4(1H)-one (noté 2-méthyl-3-benzyloxypyridin-4(1H)-one de diméthylénexylitol) (3)

Le 2-méthyl-3-benzyloxypyridin-4(*1H*)-one de diméthylènexylitol est préparé selon la méthode décrite à l'exemple 2 A partir de 5,50 g (31.4 mmol) de compose 1-amino-1-desoxy-dimethylenexylitol, on isole après recristallisation dans un mélange hexane-acétone 5.04 g (13.5 mmol) de 2-méthyl-3-benzyloxypyridin-4(*1H*)-one de diméthylènexylitol. pur d'après les analyses chromatographiques et RMN. Rendement 43%. 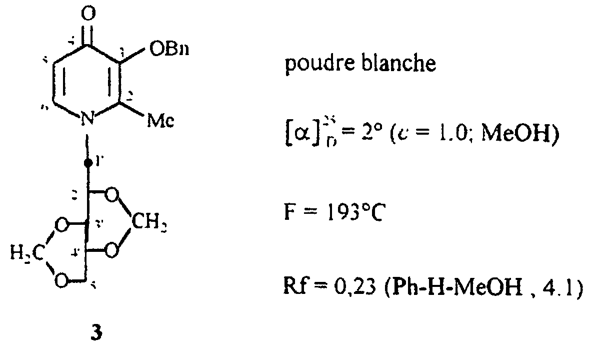 RMN ¹H sucre : δ 4,99 (dd, J_{H',H} =6,3, CH₂), 4,67 (dd, J_{H',H} =6,3, CH₂), 4,60-3,60 (massif. H-1', H-2'. H-4', H-5', H-3'),
héterocycle : δ 7,52 (d, J_{5,6} = 7,6 Hz, H-6), 7,42 - 7,31 (m, 5H Ph), 6,14 (d, J_{5,6}=7,6 Hz, H-5), 5,01 (m, CH₂-Ph), 2,21 (s, CH₃),
RMN ¹³C sucre : δ 92,0 (CH₂), 91,6 (CH₂), 75,7 (C-3'), 69,4 (C-2'), 69,4 (C-4'), 66,5 (C-5'), 52.5 (C-1'),
hétérocycle : δ 171,6 (C-4), 145,0 (C-2), 141,0 (C-3), 140,1 (C-6), 137,7 (C_{Ipso}), 128,2 (2xCₒᵣₜₕₒ), 128,1 (2xCₘₑₜₐ), 127,7 (Cₚₐᵣₐ), 115,6 (C-5), 71,7 (CH₂-Ph), 12,1 (CH₃)

### Exemple 4 Synthèse du 1-(1',2'-O-isopropylidène-α-D-xylofurano-5-yl)-2-méthyl-3-benzyloxypyridin-4(1H)-one (noté 2-méthyl-3-benzyloxypyridin-4(1H)-one demonoacétonexylose) (4) (ne fait pas partie de l'invention)

Le 2-méthyl-3-benzyloxypyridin-4(*1H*)-one de diacétonexylose est preparé selon la methode décrite à l'exemple 2 A partir de 4,50 g (23,8 mmol) de composé 5-amino-5-desoxy-monoacétonexylose, on isole après recristallisation dans un mélange dichlorométhane-éther ethylique 3.87 g (9.98 mmol) de 2-méthyl-3-benzyloxypyridin-4(*1H*)-one de monoacetonexylose, pur d'après les analyses chromatographiques et RMN. Rendement 42% 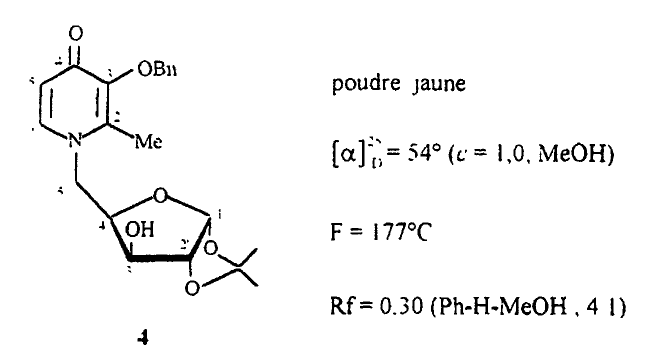 RMN ¹H sucre : δ 5,86 (d, J_{2',3'} =3.2 Hz. H-1'), 4.45 (d.J_{2',1'} =3,6 Hz, H-2'), 4,17 - 3.99 (massif. H-3'. H-4'. H-5'), 1,34 (CH₃), 1,22 (s, CH₃).
hétérocycle : δ 7,60 (d, J_{5,6} = 7,5 Hz, H-6), 7,42 - 7,27 (m, 5H Ph). 6.17 (d. J_{5,6} =7,6 Hz. H-5), 5,02 (2 dd, Hₐ CH₂-Ph), H_{b} CH₂-Ph), 2,20 (s, CH₃),
RMN ¹³C sucre :δ 110,6 (C_{Ip}), 104,3 (C-1'), 84,9 (C-2'), 79,5 (C-3'), 73,5 (C-4'), 51,6 (C-5'), 26,5 (CH₃), 25,9 (CH₃),
hétérocycle : δ 171,9 (C-4), 145,0 (C-2), 141,2 (C-3), 139,7 (C-6), 137,7 (C_{Ipso}), 128,2 (2xCₒᵣₜₕₒ), 128,1 (2xCₘₑₜₐ), 127,7 (Cₚᵤᵣₐ), 115,9 (C-5), 71,7 (CH₂-Ph), 12,2 (CH₃)

### Exemple 5 Synthèse du 1-(2',3'-O-isopropylidène-glycéryl)-2-méthyl-3-benzyloxypyridin-4(1H)-one ( noté 2-méthyl-3-benzyloxypyridin-4(1H)-one solkétal) (5)

Le 2-méthyl-3-benzyloxypyridin-4(*1H*)-one solkétal est préparé selon la méthode decrite a l'exemple 2 A partir de 5,00 g (38,1 mmol) de composé 1-amino-1-désoxy solketal, on isole après chromatographie sur gel de silice avec un mélange acétone-TEA-ethanol 80 10 10 v/v/v 5,26 g (15,9 mmol) de 2-méthyl-3-benzyloxypyridin-4(*1H*)-one solkétal, pur d'après les analyses chromatographiques et RMN. Rendement 42% 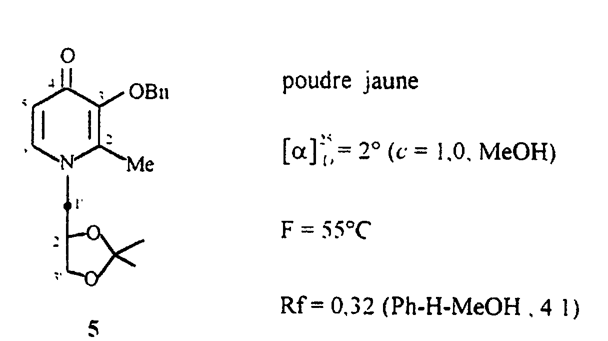 RMN ¹H sucre : δ 4,25 (m, H-2'), 4,15 - 3,89 (massif, H-1'_{a.} H-1'_{b.} H-3'). 3.59 (dd, J_{2' 3} =6.4Hz. 1,30 (s, CH₃), 1,24 (s, CH₃);
héterocycle : δ 7,55 (d, J_{5,6} = 7,4 Hz, H-6), 7,42 - 7,32 (m, 5H Ph), 6,16 (d J_{5,6}=7,6 Hz, H-5), 5,02 (2 dd, Hₐ CH₂-Ph), H_{b} CH₂-Ph), 2,19 (s, CH₃),
RMN ¹³C sucre : δ 109,1 (C_{Ip}), 74,4 (C-2'), 65,7 (C-3'), 54,6 (C-1'), 26,2 (CH₃), 25,1 (CH₃).
hétérocycle : δ 171,9 (C-4), 144,9 (C-2), 141,1 (C-3), 140,0 (C-6), 137,6 (C_{Ipso}), 128,3 (2xCₒᵣₜₕₒ), 128,1 (2xC_{méta}), 127,7 (Cₚₐᵣₐ), 115,6 (C-5), 71,7 (CH₂-Ph), 12,3 (CH₃)

### Exemple 6 Synthèse du 1-(1',2',3',4'-di-O-isopropylidène-α-D-galactopyranos-6-yl)-2-methyl-3-benzyloxypyridin-4(1H)-one (noté 2-méthyl-3-benzyloxypyridin-4(1H)-one diacétonegalactose))-one (6) (ne fait pas partie de l'invention)

Le 2-méthyl-3-benzyloxypyridin-4(*1H*)-one diacétonegalactose est préparé selon la méthode décrite à l'exemple 2 A partir de 2,5 g (9,64 mmol) de 6-amino-6-désoxy diacétonegalactose, on isole après chromatographie sur gel de silice avec un melange acétone-eau 90 10 v/v 2,03 g (4,42 mmol) de 2-méthyl-3-benzyloxypyridin-4(*1H*)-one diacétonegalactose, pur d'après les analyses chromatographiques et RMN. Rendement 46% 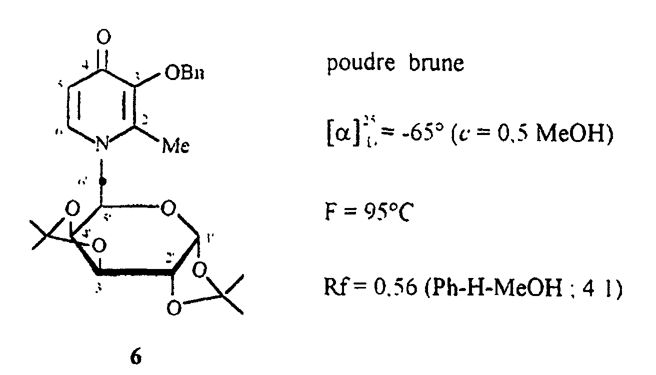 RMN ¹H sucre δ 5,40 (d, J_{1',2'} =5,0 Hz, H-1'), 4,63 (dd, J_{3',3'} =5,6 Hz, H-3'), 4,35 (dd. J_{4',5'}=2,36 Hz, H-4'), 4,27 (dd,J_{2',3'}=2,19 Hz, H-2'), 4,15 (m, H-6'ₐ), 3,96 (m, H-5'), 3,84 (dd,J_{6'a,6'b} =14,6 Hz, H-6'_{b}), 1,38 (CH₃),1,30 (s, 2xCH₃), 1,21 (s, CH₃),
héterocycle δ 7,63 (d, J_{5,6} = 7,5 Hz, H-6), 7,42 - 7,30 (m, 5H. Ph), 6,16 (d. J_{5 6} = 7,6 Hz, H-5), 4,99 (2 dd, J_{Ha,Hb}=11,1, H_{a CH2-Ph}, H_{b CH2-Ph}), 2,18 (s, CH₃),
RMN ¹³C sucre δ 108,7 (C_{Ip}), 107,9 (C_{Ip}), 95,5 (C-1'), 70,3 (C-2'), 70,1 (C-3'), 69,6 (C-4'), 67,2(C-5'), 52,2 (C-6'), 25,9 (CH₃), 25,6 (CH₃), 24,6 (CH₃), 24,2 (CH₃),
héterocycle δ 171,9 (C-4), 144,9 (C-2), 141,2 (C-3), 139,7 (C-6), 137,7 (C_{Ipso}), 128,2 (2xCₒᵣₜₕₒ), 128,0 (2xC_{méta}), 127,7 (Cₚₐᵣₐ), 115,7 (C-5), 71,8 (CH₂-Ph), 12,1 (CH₃)

### Exemple 7 Synthèse du 1-(2',3':5',6'-di-O-isopropylidène-α-D-mannofuranoside de but-4-yl)-2-méthyl-3-benzyloxypyridin-4(1H)-one (noté 2-méthyl-3-benzyloxypyridin-4(1H)-one de diacetonemannoside de butyle) (7) (ne fait pas partie de l'invention)

Le 2-methyl-3-benzyloxypyridin-4(*1H*)-one de diacétonemannoside de butyle est prépare selon la méthode décrite à l'exemple 2 A partir de 2,00 g (6.04 mmol) de diacetonemannoside de 4'-aminobutyle , on isole après chromatographie sur gel de silice avec un mélange acetone-eau 90 10 v/v 1.72 g (3.25 mmol) de 2-méthyl-3-benzyloxypyridin-4(*1H*)-one de diacetonemannoside de butyle, pur d'après les analyses chromatographiques et RMN. Rendement 54% 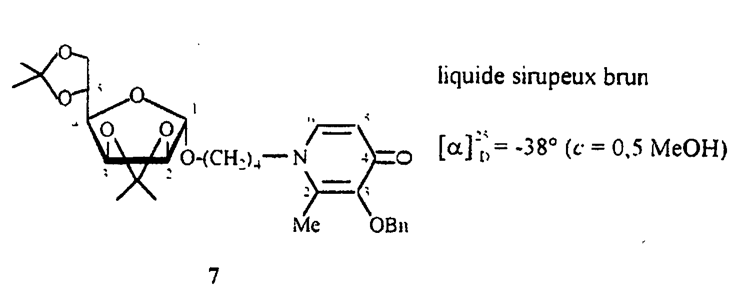 RMN ¹H sucre : δ 4,95 (s, H-1'), 4,73 (dd, J_{3,4}=3,5 Hz, H-3'), 4,52 (d, J_{2.3}=5,8 Hz, H-2). 4.26 (dd, J_{5,6a} = 6,1 Hz, H-5'), 4,01 (dd, J_{6a,6b} = 8,4 Hz, J_{5,6b} = 6,8 Hz, H-6ₐ), 3,88-3,82 (m, H-6_{b}, H-4',H-4"), 3,54 (dd, J_{1"a,1"b} = 7,1 Hz,H-1"ₐ), 3,43 (dd, J_{1"a,2"} = 3,9 Hz, H-1'_{b}), 1,59-1,45 ( m, H-2', H-3'), 1,35 (s, CH₃), 1,32 (s, CH₃), 1,26 (s, CH₃), 1,24 (s, CH₃).
hetérocycle : δ 7,56 (d, J_{5,6} = 7,4 Hz, H-6), 7,41 - 7,30 (m, 5H. Ph), 6,14 (d, J_{5,6} = 7,5 Hz, H-5), 5,03 ( m, CH₂-Ph), 2,14 (s, CH₃),
RMN ¹³C sucre : δ 111,5 (C_{Ip}), 108,0 (C_{Ip}), 105,6 (C-1'), 84,3 (C-2'), 79,7 (C-4'), 78.9 (C-3'), 72,4 (C-5'), 66,0 (C-1"), 65,9 (C-6'), 52,3 (C-4"), 26,9 25,6 (C-2", C-3"), 26,4 (CH₃) 25,6 (CH₃), 25,0 (CH₃), 24,2 (CH₃)
hétérocycle : δ 171,7 (C-4), 145,2 (C-2), 140,3 (C-3), 139,2 (C-6), 137,7 (C_{Ipso}). 128,3 (2xCₒᵣₜₕₒ), 128,0 (2xCₘₑₜₐ), 127,6 (Cₚₐᵣₐ), 115,9 (C-5), 71,6 (CH₂-Ph), 11,7 (CH₃)

### Exemple 8 Synthèse du 1-glycéryl-2-méthyl-3-benzyloxypyridin-4(1H)-one (noté 2 méthyl-3-benzyloxypyridin-4(1H)-one glycérol) (8)

35,6 g (165 mmol, 1,5 éq) de 2-méthyl-3-benzyloxy-4-pyranone, 10 g (110 mmol) de 1-amino-1-desoxy-glycérol sont introduits dans 600 mL d'un mélange éthanol-eau 50 50 puis on ajoute 14 mL d'une solution aqueuse de soude (2N) Le milieu réactionnel est alors porte sous agitation au reflux pendant 48 heures On laisse refroidir le milieu réactionnel puis on ajoute une solution de HCl concentré jusqu'à pH=1 Après évaporation de l'ethanol on extrait le résidu par de l'éther éthylique (3 x 100 mL) afin d'éliminer l'excès de 2-méthyl-3-benzyloxy-4-pyranone La phase aqueuse est amenée à neutralité par addition d'une solution aqueuse de NaOH puis concentrée sous pression réduite. Le résidu est repris par un minimum d'eau On isole par précipitation 12,7 g (43,9 mmol) de 2-methyl-3-benzyloxypyridin-4(1H)-one de glycérol, pur d'après les analyses chromatographiques et RMN Rendement 40% 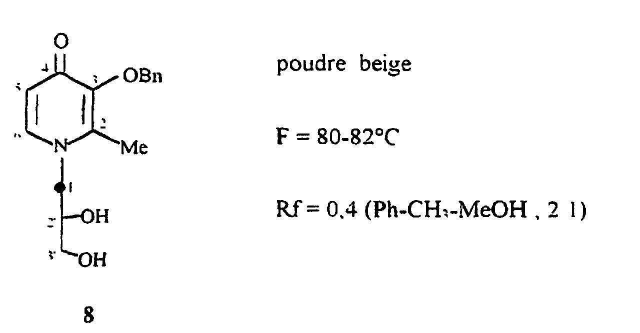 RMN ¹H sucre : δ 3,93 (d,J_{1'a,1'b}=12,1 Hz, H-1'ₐ), 3,72 (d, H-1'_{b}), 3,79 (massif, H-2'). 3,48 (m, H_{3'a}, H_{3'b}).
hétérocycle : δ 7,38 (d, J_{5,6} = 7,15 Hz, H-6), 7,26 - 7,09 (m, 5H Ph), 6,20 (d, J_{5,6} = 7.15 Hz, H-5), 4,93 (d, J_{Ha,Hb}=12,1, Hₐ CH₂-Ph), 4,84 (d, H_{b} CH₂-Ph), 2,06 (s, CH₃).
RMN ¹³C sucre : δ 70,4 (C-2'), 63,8 (C-3'), 56,8 (C-1')
héterocycle : δ 171,9 (C-4), 144,9 (C-2), 141,1 (C-3), 140,0 (C-6), 137,6 (C_{Ipso}).
128,8 (2xCₘₑₜₐ), 128,3 (2xCₒᵣₜₕₒ), 128,2 (Cₚₐᵣₐ), 115,9 (C-5), 73,3 (CH₂-Ph), 12,7 (CH₃)

### Exemple 9 Synthèse du 1-(2',4' 3',5'-di-O -méthylène-xylityl)-2-méthyl-3-hydroxypyridin-4(1H)-one (noté 2-méthyl-3-hydroxypyridin-4(1H)-one de diméthylènexylitol) (9)

1.00 g (2,68 mmol) de 2-méthyl-3-benzyloxypyridin-4(*1H*)-one de dimethylènexylitol sont introduits dans le mélange 10 mL éthanol - 2 mL H₂O et l' on ajoute 0.11 g de Pd/C à 10%. Le milieu réactionnel est soumis à l'hydrogenolyse catalytique sous agitation à température ambiante pendant 4 heures A la fin de cette hydrogenolyse. on filtre le milieu réactionnel La solution est évaporée sous pression réduite et on isole après extraction et recristallisation dans un mélange hexane-acétone 589 mg (2.09 mmol) de 2-methyl-3-hydroxypyridin-4(*1H*)-one de diméthylènexylitol, pur d'après les analyses chromatographiques et RMN. Rendement 78% 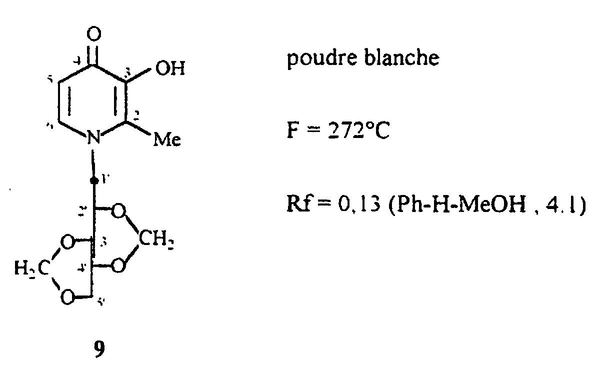 RMN ¹H sucre : δ 5,01 (dd, J_{H',H} =6,3, CH₂), 4,69 (dd, J_{H',H} =6,3, CH₂), 4,16-3,62 (massif, H-1', H-2', H-4', H-5', H-3'),
hétérocycle : δ 7,48 (d, J_{5,6} = 7,3 Hz, H-6), 6,10 (d, J_{5,6} =7,3 Hz, H-5), 2,21 (s, CH₃),
RMN ¹³C sucre : δ 92,0 (CH₂), 91,8 (CH₂), 75,9(C-3'), 69,4 (C-2'), 69,4 (C-4'), 68,5 (C-5'), 52,6 (C-1'),
hétérocycle : δ 170,0 (C-4), 145,1 (C-2), 138,4 (C-6), 128,9 (C-3), 110,3 (C-5), 11,5 (CH₃)
IR(KBr) 3147, 1627 cm⁻¹
UV (MeOH) λₘₐₓ = 298 nm, log ε = 3,04

### Exemple 10 Synthèse du 1-(2',3' 4',5'-di-O-isopropylidène-xylityl)-2-methyl-3- hydroxypyridin-4(1H)-one (noté 2-méthyl-3-hydroxypyridin-4(1H)-one de diacetonexylitol) (10)

Le 2-méthyl-3-hydroxypyridin-4(*1H*)-one de diacétonexylitol est préparé selon la methode décrite dans l'exemple 9 A partir de 1,50 g (3,49 mmol) de 2-méthyl-3-benzyloxypyridin-4(*1H*)-one de diacétonexylitol on isole après extraction et recristallisation dans le mélange hexane- acétone 604 mg (1.78 mmol) de 2-méthyl-3-hydroxypyridin-4(*1H*)-one de diacétonexylitol, pur d'après les analyses chromatographiques et RMN Rendement 51% 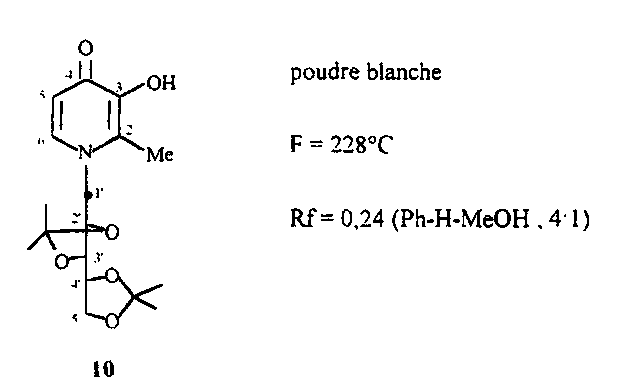 RMN ¹H sucre : δ 4,23-4,05 (massif, H-1'_{a,} H-1'_{b,} H-2', H-4', H-5'ₐ), 3,84 (dd. J_{3',4'} = 7,6, Hz, J_{2',3'} = 7, H-3'), 3,72 (dd, H-5'_{b}), 1,33 (s, CH₃), 1,31 (s, CH₃), 1,29 (s, CH₃), 1.29 (s, CH₃),
heterocycle : δ 7,54 (d, J_{5,6} = 7,6 Hz, H-6), 7,42 - 7,31 (m, 5H Ph), 6,21 (d, J_{5,6} = 7,6 Hz, H-5), 5,02 (2 dd, Hₐ CH₂-Ph), H_{b} CH₂-Ph), 2,20 (s, CH₃),
RMN ¹³C sucre : δ 109,4 (C_{Ip}), 108,7 (C_{Ip}), 77,5 (C-3'), 76,1 (C-2'), 73,9 (C-4'), 64,8 (C-5'), 54,2 (C-1'), 26,7 (CH₃), 26,5 (CH₃), 25,9 (CH₃), 25,3 (CH₃),
hétérocycle : δ 171,9 (C-4), 144,9 (C-2), 141,0 (C-3), 140,0 (C-6), 137.6 (C_{Ipso}), 128,4 (2xCₒᵣₜₕₒ), 128,1 (2xCₘₑₜₐ), 127,7 (Cₚₐᵣₐ), 115,7 (C-5), 71,7 (CH₂-Ph), 13,3 (CH₃)
IR ( KBr ) 3183, 1626 cm⁻¹
UV (MeOH) λₘₐₓ = 299 nm, log ε = 3,04

### Exemple 11 Synthèse du 1-(2',3'-O-isopropylidène-glycéryl )-2-méthyl-3-hydroxypyridin-4(1H)-one (noté 2-méthyl-3-hydroxypyridin-4(1H)-one de solkétal) (11)

Le 2-méthyl-3-hydroxypyridin-4(*1H*)-one de solkétal est préparé selon la méthode décrite dans l'exemple 9. A partir de 1.00 g (3.04 mmol) de 2-méthyl-3-benzyloxypyridin-4(*1H*)-one de solkétal, on isole après extraction et recristallisation dans un mélange hexane-acétone 545 mg (2,28 mmol) de 2-méthyl-3-hydroxypyridin-4(*1H*)-one de solkétal, pur d'après les analyses chromatographiques et RMN Rendement 75% 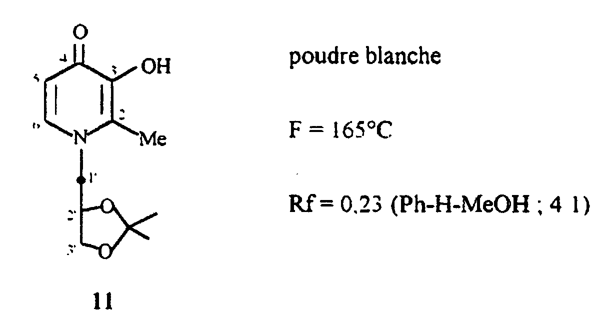 RMN ¹H sucre : δ 4.30 (m, H-2'), 4,21 - 3,93 (massif, H-1'_{a,} H-1'_{b,} H-3'ₐ), 3.65 (dd, J_{2',3'} = 6,2 Hz), 1,32 (s, CH₃), 1,24 (s, CH₃);
hétérocycle : δ 7,55 (d, J_{5,6} = 7,4 Hz, H-6), 7,42 - 7,32 (m, 5H Ph), 6,16 (d, J_{5,6} = 7,6 Hz. H-5), 5,02 (2 dd, Hₐ CH₂-Ph), H_{b} CH₂-Ph), 2,19 (s, CH₃),
RMN ¹³C sucre : δ 109,1 (C_{Ip}), 74,5 (C-2'), 65,6 (C-3'), 54,7 (C-1'), 26,2 (CH₃), 25.1 (CH₃),
hétérocycle : δ 170,0 (C-4), 145,1 (C-2), 139,2 (C-6), 129,1 (C-3), 110,4 (C-5), 11,7 (CH₃)
IR ( KBr ) 3130, 1630 cm⁻¹
UV (MeOH) λₘₐₓ = 298 nm, log ε = 3,03

### Exemple 12 Synthèse du 1-(1',2'-O-isopropylidène-a-D-xylofuranos-5-yl)-2-méthyl-3-hydroxypyridin-4-(1H)-one (noté 2-méthyl-3-hydroxypyridin-4-(1H)-one de monoacetonexylose) (12) (ne fait pas partie de l'invention)

Le 2-méthyl-3-hydroxypyridin-4-(*1H*)*-*one de monoacétonexylose est préparé selon la methode décrite à l'exemple 9. A partir de 1,00 g (2,58 mmol) de 2-méthyl-3-benzyloxypyridin-4-(*1H*)-one de monoacétonexylose, on isole après extraction et recristallisation dans un mélange hexane-acétone 545 mg (1,83 mmol) de 2-méthyl-3-hydroxypyridin-4-(*1H*)-one de monoacétonexylose, pur d'après les analyses chromatographiques et RMN Rendement 71% 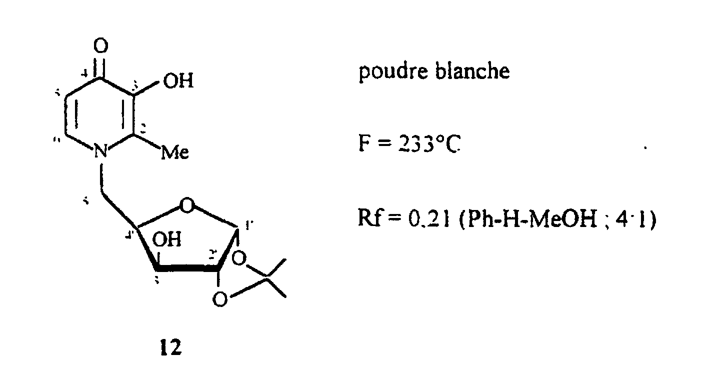 RMN ¹H sucre : δ 5,87 (d,J_{1',2'} =3,5 Hz, H-1'), 4,46 (d,J_{2',3'}=3,8 Hz, H-2'), 4,22 - 4,05 (massif, H-3' H-4', H-5'), 1,33 (CH₃), 1,21 (s, CH₃),
héterocycle : δ 7,57 (d, J_{5 6} = 9,3 Hz, H-6), 6,14 (d, H-5), 2,30 (s, CH₃),
RMN ¹³C sucre : δ 110,7 (C_{Ip}), 104,3 (C-1'), 84,7 (C-2'), 79,6 (C-3'), 73,5 (C-4'), 51,6 (C-5'), 26,5 (CH₃), 25,9 (CH₃),
héterocycle : δ 169,9 (C-4), 145,1 (C-2), 139,9 (C-6), 129,2 (C-3), 110,7 (C-5), 12,2 (CH₃)
IR ( KBr ) 3178, 1635 cm⁻¹
UV (MeOH) λₘₐₓ = 298 nm, log ε = 3,04

### Exemple 13 Synthèse du 1-(1',2' 3',4'-di-O-isopropylidène-α-D-galactopyranos-6-yl)-2-methyl-3-hydroxypyridin-4(1H)-one ( noté 2-méthyl-3-hydroxypyridin-4(1H)-one diacetonegalactose ) (13) (ne fait pas partie de l'invention)

Le 2-méthyl-3-hydroxypyridin-4(*1H*)-one diacétonegalactose est préparé selon la méthode décrite dans l'exemple 9 A partir de 900 mg (1,97 mmol) de 2-méthyl-3-benzyloxypyridin-4(*1H*)-one diacétonegalactose, on isole après extraction et recristallisation dans un mélange hexane-acétone 521 mg (1,42 mmol) de 2-méthyl-3-hydroxypyridin-4(*1H*)-one diacétonegalactose, pur d'après les analyses chromatographiques et RMN Rendement 72% 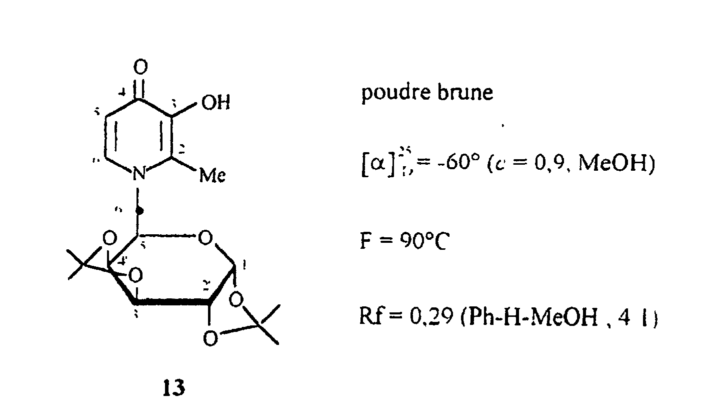 RMN ¹H sucre : δ 5,41 (d, J_{1',2'} =4,9 Hz, H-1'), 4,64 (dd, J_{3',4'} =7,7 Hz, H-3'), 4,33 (dd, J_{4'5'} =7,7 Hz H-4'), 4,25 (dd, J_{2',3'} =2,19 Hz, H-2'), 4,08 (m, H-6'ₐ), 3,98 (m, H-5'), 3,87 (dd, H-6'_{b}), 1,38 (CH₃),1,29 (s, 2xCH₃), 1,22 (s, CH₃),
hétérocycle : δ 7,61 (d, J_{5,6} = 7,3 Hz, H-6), 6,15 (d, J_{5,6} = 7,4 Hz, H-5), 2,22 (s CH₃),
RMN ¹³C sucre : δ 108,7 (C_{Ip}), 107,9 (C_{Ip}), 95,5 (C-1'), 70,4 (C-2'), 70,1 (C-3'), 69,5 (C-4'), 67,2 (C-5'), 52,3 (C-6'), 25,9 (CH₃), 25,6 (CH₃), 24,6 (CH₃), 24,3 (CH₃),
hétérocycle : δ 168,7 (C-4), 145,3 (C-2), 137,8 (C-6), 129,8 (C-3), 110,5 (C-5), 11,6(CH₃)
IR ( KBr ) 3176, 1632 cm⁻¹
UV (MeOH) λₘₐₓ = 298 nm, log ε = 3,04

### Exemple 14 Synthèse du 1-glycéryl-2-méthyl-3-hydroxypyridin-4(1H)-one (noté 2 methyl-3-hydroxypyridin-4(1H)-one glycérol) (14)

4.2 g (14,5 mmol) de 2-méthyl-3-benzyloxypyridin-4(*1H*)-one de glycérol sont introduits dans le mélange 54 mL éthanol - 6 mL H₂O et l'on ajoute 0.11 g de Pd/C à 10° Le milieu reactionnel est soumis à l'hydrogénolyse catalytique sous agitation à température ambiante pendant 24 heures A la fin de cette hydrogenolyse, on filtre le milieu reactionnel Le Pd/C filtré est lavé par EtOH puis le filtrat est évaporée sous pression réduite Le résidu solide est recristallisé dans un mélange MeOH-acétate d'éthyle pour conduire a 2,05 g (10.3 mmol) de 2-méthyl-3-hydroxypyridin-4(*1H*)-one de glycérol, pur d'après les analyses chromatographiques et RMN Rendement 71% 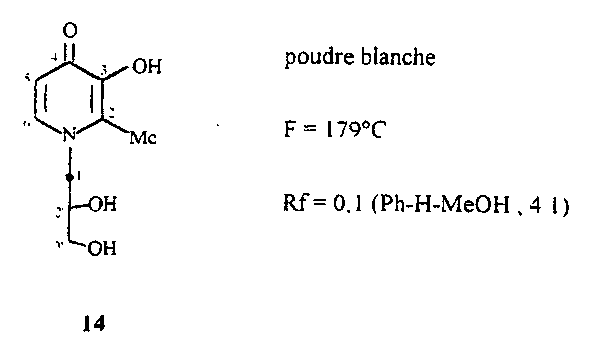 RMN ¹H sucre : δ 4.15 (d,J_{1'a,1'b}=13,7Hz, H-1'ₐ), 3,74 (dd, J_{1'b,2} =8,7Hz H-1'_{b}), 3 67 (massif, H-2'), 3,42 (dd, J_{3'a,3'b}=10,7Hz, J_{3'a,2}=4,2Hz, H_{3'a}), 3,30 (dd, J_{3'b 2'}=6,4Hz, H_{3'b}).,
hétérocycle : δ 7,47 (d, J_{5,6} = 7,35 Hz, H-6), 6,11 (d, H-5), 2,30 (s, CH₃),
RMN ¹³C sucre : δ 70,6 (C-2'), 63,2 (C-3'), 55,7 (C-1'),
hétérocycle : δ 168,8 (C-4), 145,1 (C-2), 138,5 (C-6), 129,3 (C-3), 110,1 (C-5), 11,6 (CH₃)
IR(KBr) 3130, 1630 cm⁻¹
UV (MeOH) λₘₐₓ = 298 nm, log ε = 3,03

### Exemple 15 Synthèse du 1-(2',4' 3',5'-di-O-méthylène-xylityl)-2-methyl-3-hydroxypyridin-4(1H)-one (noté 2-méthyl-3-hydroxypyridin-4(1H)-one de dimethylenexylitol) (9) par hydrogénolyse en milieu acide

1.00 g (2.68 mmol) de composé 2-méthyl-3-benzyloxypyridin-4(*1H*)-one de dimethylenexylitol sont introduits dans le mélange 10 mL éthanol - 2 mL H₂O, on ajoute 0.08 g de Pd/C à 10% et le pH de la solution est ajusté à pH=1 avec une solution de HCl concentre Le milieu réactionnel est alors soumis à l'hydrogénolyse catalytique sous agitation a température ambiante pendant 4 heures A la fin de cette hydrogénolyse, on filtre le milieu reactionnel On ajoute 25 mL d'eau au résidu et la solution est neutralisée par addition d'une solution aqueuse de soude On extrait le milieu réactionnel par 2x20 mL de dichlorométhane Les phases organiques sont rassemblées, séchées sur sulfate de sodium. filtrees et évaporées sous pression réduite On isole 509 mg (1,80 mmol) de 2-méthyl-3-hydroxypyridin-4(*1H*)-one de diméthylènexylitol pur d'après les analyses chromatographiques et RMN. Rendement 67%

Les constantes physiques sont identiques à celles décrites dans l'exemple 9

### Exemple 16 Synthèse du 1-glycéryl-2-méthyl-3-hydroxypyridin-4(1H)-one (noté 2 methyl-3-hydroxypyridin-4(1H)-one glycérol) (14) par hydrogénolyse en milieu acide

Le 2-méthyl-3-hydroxypyridin-4(*1H*)-one glycérol est préparé à partir du 2-méthyl-3-hydroxypyridin-4(*1H*)-one solkétal selon la méthode décrite dans l'exemple 15 Le protocole d'extraction et de purification est le suivant filtration du catalyseur, addition d'eau puis élimination du solvant organique, lyophilisation, reprise du résidu par un solvant organique, neutralisation par résine Dowex OH⁻, filtration puis évaporation du solvant A partir de 1,00 g (3,04 mmol) de 2-méthyl-3-benzyloxypyridin-4(*1H*)-one solkétal, on isole 790 mg (2,73 mmol) de 2-méthyl-3-hydroxypyridin-4(*1H*)-one glycérol, pur d'après les analyses chromatographiques et RMN. Rendement 90%.
Les constantes physiques sont identiques à celles décrites dans l'exemple 14

### Exemple 17 Synthèse du 1-xylityl-2-méthyl-3-hydroxypyridin-4(1H)-one (noté 2 methyl-3-hydroxypyridin-4(1H)-one xylitol) (17) par hydrogénolyse en milieu acide

Le 2-methyl-3-hydroxypyridin-4(*1H*)-one xylitol est préparé à partir du 2-méthyl-3-hydroxypyridin-4(*1H*)-one diacétonexylitol (2) selon la méthode décrite dans l'exemple 16 A partir de 10,1 g (23.5 mmol) de 2-méthyl-3-benzyloxypyridin-4(*1H*)-one diacetonexylitol, on isole 4.25 g (16.3 mmol) de 2-méthyl-3-hydroxypyridin-4(*1H*)-one xylitol, pur d'après les analyses chromatographiques et RMN Rendement 69% 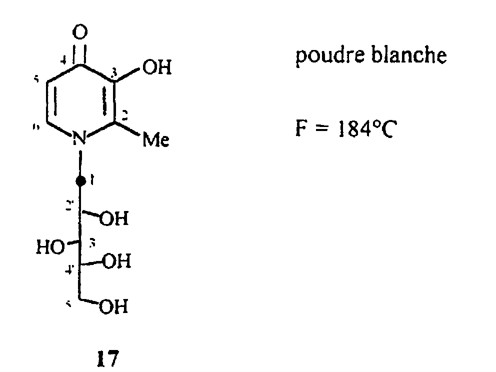 RMN ¹³C sucre : δ 71,2-70,9 (C-2'- C-4'), 62,4(C-5'), 55,7 (C-1')
héterocycle : δ 168,8 (C-4), 144,9 (C-2), 141,0 (C-3), 138,4 (C-6), 110,1 (C-5), 11,6 (CH₃)

### Exemple 18 Complexation avec Fe^{III}

L'étude de la complexation vis-à-vis de Fe^{III} des différents ligands a été étudiée sur un spectrophotomètre JASCO V-530 piloté via un micro-ordinateur doté de programmes d'acquisition et d'analyse Les propriétés chélatantes des 2-méthyl-3-hydroxypyridin-4(*1H*)-one solkétal (11), 2-méthyl-3-hydroxypyridin-4(*1H*)-one glycérol (14). 2-méthyl-3-hydroxypyridin-4(*1H*)-one de diacétonexylitol (10) et 2-méthyl-3-hydroxypyridin-4(*1H*)-one de xylitol (17) et du 2-méthyl-3-hydroxypyridin-4(*1H*)-one de monoacetonexylose (12) ont ete comparées à celles du 1,2-diméthyl-3-hydroxypyridin-4(*1H*)-one (noté Déferiprone ou L1) en utilisant une méthode spectrophotométrique UV

Les echantillons ont été preparés comme suit
- préparation de solutions de ligands dans H₂O à la concentration de 3, 6 10⁻³ M.
- preparation d'une solution aqueuse de Fe^{III} à 1.2 10⁻³ M à partir de FeCl₃,6H₂O

La gamme d'échantillons est obtenue par mélange des deux solutions de ligands dans les proportions suivantes

| | | |
|---|---|---|
| 1 mL de FeCl₃ à 1,2 10⁻³ M | + 0,2 mL de ligand à 3, 6 10⁻³ M | + 0.8 mL d'H₂O |
| 1 mL " " | + 0,6 mL " " | + 0,4 mL " |
| " " " | + 0,7 mL " " | + 0.3 mL " |
| " " " | + 0,8 mL " " | + 0,2 mL " |
| " " " | + 1 mL " " | |

Ainsi la gamme d'échantillon obtenue présente respectivement les valeurs de Rv suivantes 0,2 , 0,4 , 0,6 , 0,7; 0,8 , I La solubilité du 2-méthyl-3-hydroxypyridin-4(*1H*)-one de diacétonexylitol (10) dans l'eau étant faible, sa complexation vis-à-vis de Fe^{III} a ete évaluée à partir d'une solution aqueuse de chlorhydrate à 3,6 10⁻³ M Cette dernière a été obtenue par addition d'une quantité stoechiométrique de HCl.

Les spectres des solutions de ligand d'une part, de FeCl₃ d'autre part, présentent un seul pic d'absorption et sont caractérisés par
- Défériprone® et dérivés 10-12, 14 et 17 λₘₐₓ =293-296 nm;
- FeCl₃ λₘₐₓ 302 nm

La chélation est instantanée et se traduit par l'apparition d'une coloration violette des le mélange des deux solutions Les solutions restent limpides et aucune précipitation n'est observée

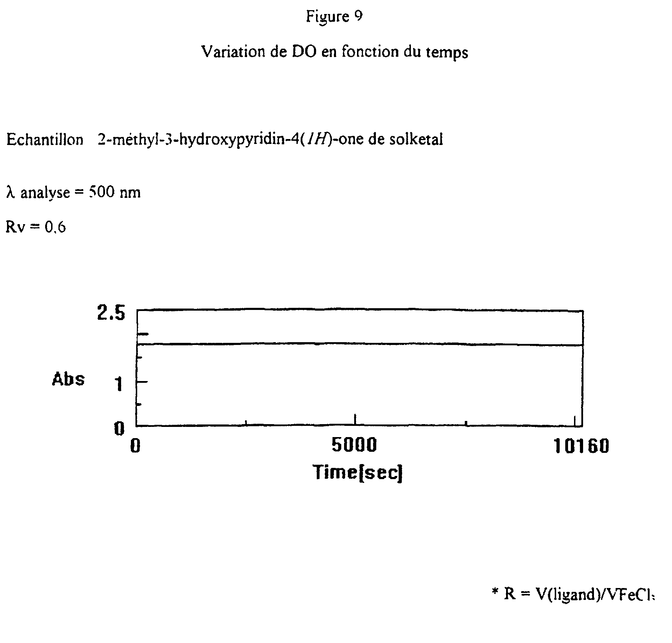

Un contrôle de la DO au cours du temps (planche hors texte, figure 9) souligne la stabilite du complexe ainsi que sa formation immédiate et quantitative

Les spectres des différents mélanges indiquent l'apparition d'un maximum d'absorption a un λₘₐₓ voisin de 500 nm.

Quelle que soit la nature du ligand, la courbe DO = f(Rv) obtenue est assimilable a une droite dans la gamme de R compris entre 0 et 0,7 (planches hors texte , figures 1 à 8)

A l'exception du 2-méthyl-3-hydroxypyridin-4(*1H*)-one de xylitol (17) (planche hors texte n°8/16), au-delà du rapport Rv = 0,7, la courbe évolue vers une asymptote avec des valeurs de DO généralement supérieures à 1,5

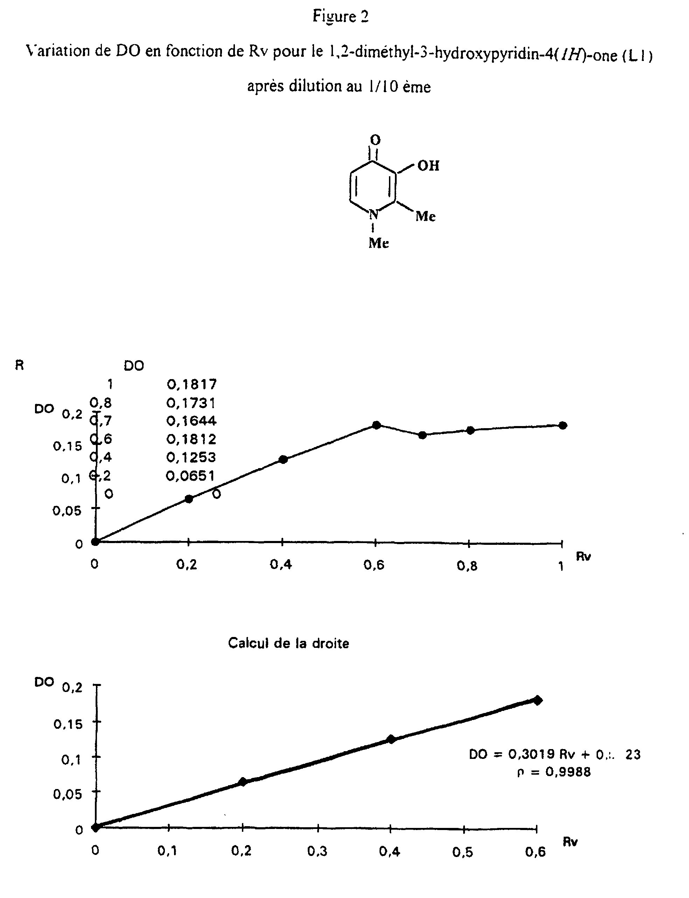

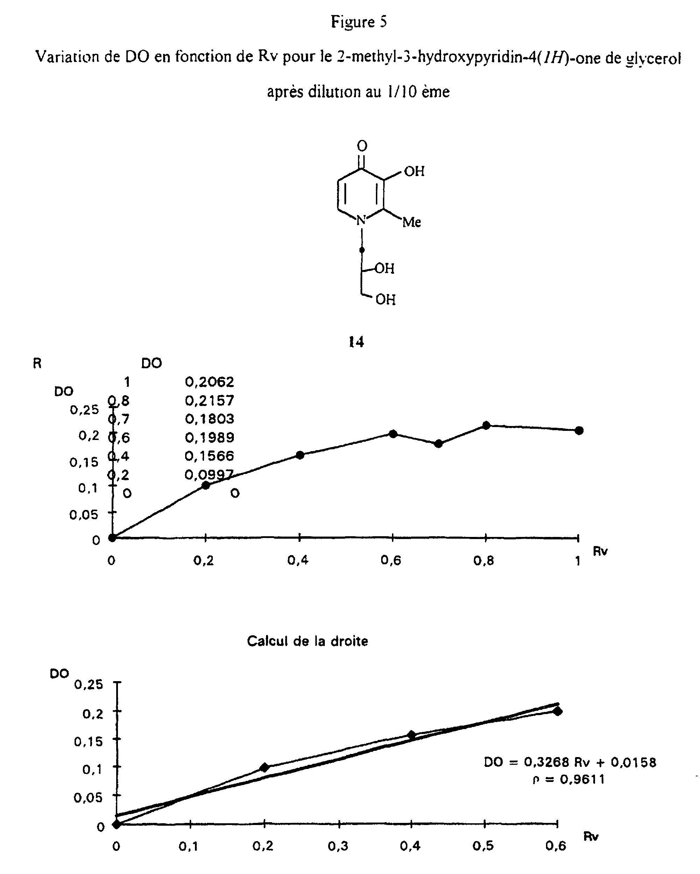

Ce palier n'est pas imputable aux valeurs de DO élevées constatées au-delà de Rv = 0.6 car le diagramme obtenu après dilution des échantillons au 1/10 est identique aux valeurs de DO près, conserve le même aspect (exemples du L₁ (planche hors texte. figure 2) et 2-méthy]-3-hydroxypyridin-4(*1H*)-one de glycérol(14) (planche hors texte, figure 5))

Ce phénomène asymptotique n'est pas constaté pour 2-méthyl-3-hydroxypyridin-4(*1H*)-one de xylitol qui présente une variation linéaire pour l'ensemble des valeurs de Rv allant de 0 à 1 Les équations des droites obtenues pour l'ensemble des produits sont rassemblées dans le tableau ci-après

| Ligand | Equation de droite | Domaine de. calcul |
|---|---|---|
| 1,2-dimethyl-3-hydroxypyridin-4(*1H*)-one | DO=3.034 Rᵥ- 0.027 ρ =0.995 | 0 < Rᵥ < 0.6 |
| 2-methyl-3-hydroxypyridin-4(*1H*)-one de diacétonexylitol **(10)** | DO = 3.125 Rv+0.035 ρ =0.984 | 0 < Rᵥ < 1 |
| 2-methyl-3-hydroxypyridin-4(*1H*)-one de xylitol **(17)** | DO = 1.996 Rᵥ + 0.044 ρ=0.992 | 0 < Rᵥ < 1 |
| 2-methyl-3-hydroxypyridin-4(*1H*)-one de solkétal **(11)** | DO = 3 189 Rᵥ - 0.110 ρ = 0.988 | 0 < Rᵥ < 0.7 |
| 2-methyl-3-hydroxypyridin-4(*1H*)-one de glycérol **(14)** | DO = 3.315 Rᵥ - 0.032 ρ=0.996 | 0 < Rᵥ < 0.6 |
| 2-methyl-3-hydroxypyridin-4(*1H*)-one **(12)** de monoacétonexylose | DO = 2.943 Rᵥ + 0.010 ρ =0.996 | 0 < Rᵥ < 1 |

### Exemple 19 Toxicité aigue chez la Souris

Les toxicités aigues des composés **10-12, 14** et **17** ainsi que celles du L1 ont été évaluées chez la Souris.

Les échantillons sont mis en solution soit dans l'eau, soit dans le mélange eau-DMSO (3 2 , v/v) de façon à obtenir des solutions de concentrations variables inférieures ou égales à 130 g.L⁻¹ Les volumes injectés sont calculés sur la base de 0,2 mL pour un poids de souris de 20 g.

Le 1,2-diméthyl-3-hydroxypyridin-4(*1H*)-one (L1) a été solubilisé sous forme de chlorhydrate La toxicité est évaluée à partir de solutions aqueuses tamponnées ou non par NaH₂PO₄ ou encore par Na₂HPO₄, ou encore après addition d'une quantite stoechiométrique de NaHCO₃

Les dérivés 2-méthyl-3-hydroxypyridin-4(*1H*)-one de glycérol **(14),** de xylitol **(17),** de monoacétonexylose **(12)** et de solkétal **(11)** ont été injectés par voie 1 V

Le composé 2-méthyl-3-hydroxypyridin-4(*1H*)-one de diacétonexylitol **(10)** a ete injecte par voie I. P

Les résultats rassemblés dans le tableau montrent que ,
- le 2-méthyl-3-hydroxypyridin-4(*1H*)-one de glycérol **(14),** qui presente une solubilité supérieure à 130 g.L⁻¹ dans l'eau, est atoxique à 1300 mg.kg⁻¹ contrairement au L qui induit une mortalité de 100 % à cette dose,
- le 2-méthyl-3-hydroxypyridin-4(*1H*)-one de solkétal **(11),** présente une toxicité supérieure à celle du L 1 et induit des troubles neurophysiologiques,
- le 2-méthyl-3-hydroxypyridin-4(*1H*)-one de xylitol **(17),** et le 2-méthyl-3-hydroxypyridin-4(*1H*)-one monoacétonexylose **(12)** présentent les premiers effets toxiques a 1300 mg kg⁻¹ (² DL10) et s'avèrent donc moins toxiques que le L1.
- le 2-méthyl-3-hydroxypyridin-4(*1H*)-one de diacétonexylitol **(10)** présente une toxicité plus faible que L1 mais est moins soluble aux doses étudiées

| Composés | Sexe | Toxicté aigue chez la Souris DL50 ± S E |
|---|---|---|
| L1 chlorydrate 1,2-diméthyl-3-hydroxypyridin-4(*1H*)-one | M | 783 ± 50 mg.kg⁻¹ 868 ± 86 mg.kg⁻¹ |
| | F | |
| 2-methyl-3-hydroxypyridin-4(*1H*)-one de glycerol (14) | M | innocuité totale à 1300 mg.kg⁻¹ (solubilité maximum ) |
| | F | |
| 2-methyl-3-hydroxypyridin-4*(1H)*-one de xylitol (17) | M | innocuité totale à 1000 mg.kg⁻¹ (solubilité maximum ) |
| | F | |
| 2-méthyl-3-hydroxypyridin-4(*1H*)-one de monoacétonexylose (12) | M | innocuité totale à 1300 mg kg⁻¹ |
| | F | DL 10 = 1000 mg.kg⁻¹ |
| 2-methyl-3-hydroxypyridin-4(*1H*)-one de solkétal (11) | M | 817 ± 75 mg kg⁻¹ |
| | F | 745 ± 84 mg kg⁻¹ |
| 2-methyl-3-hydroxypyridin-4(*1H*)-one de diacétonexylitol (10) | M | > 650mg kg⁻¹ (I. P ) |
| | F | |

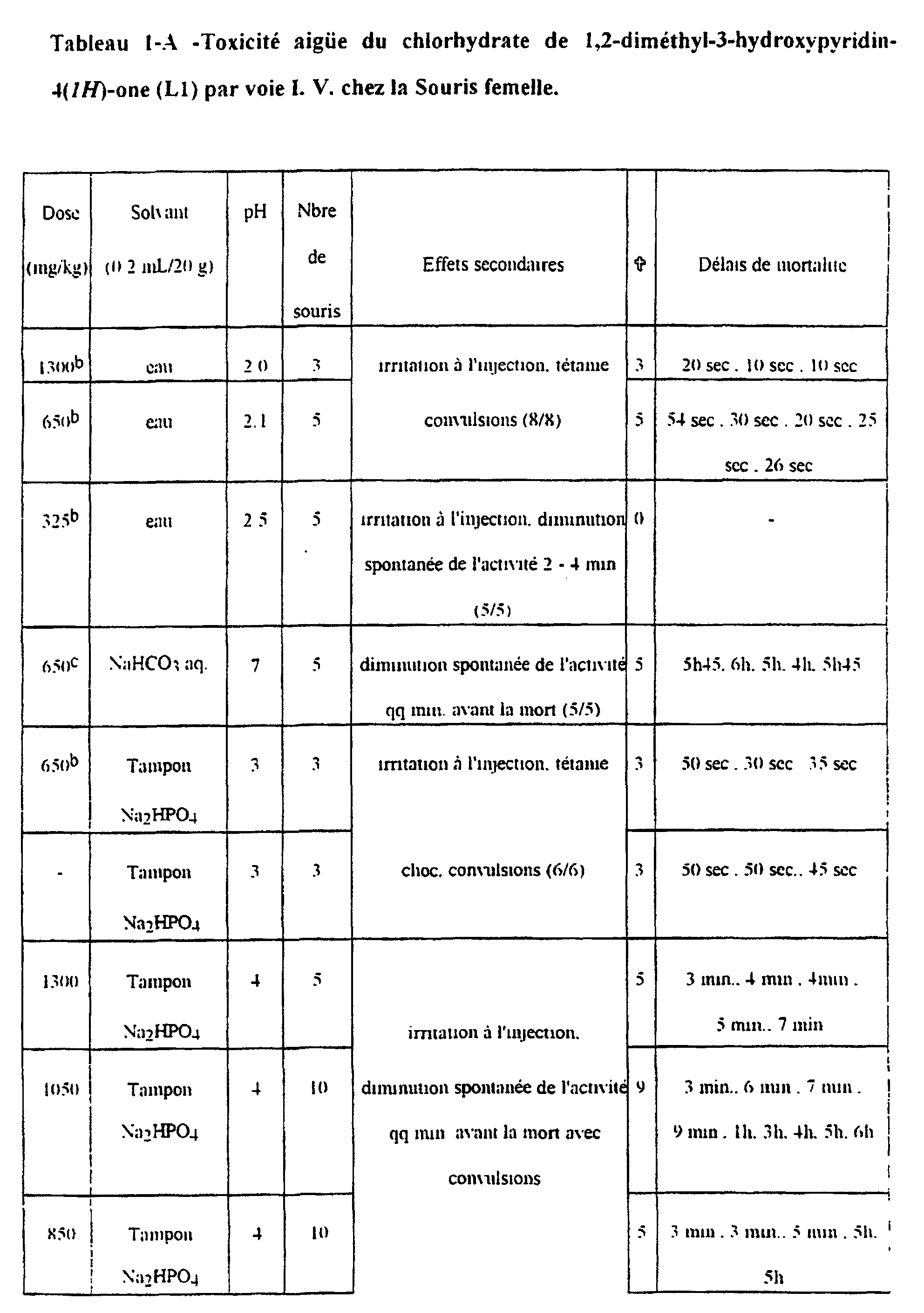

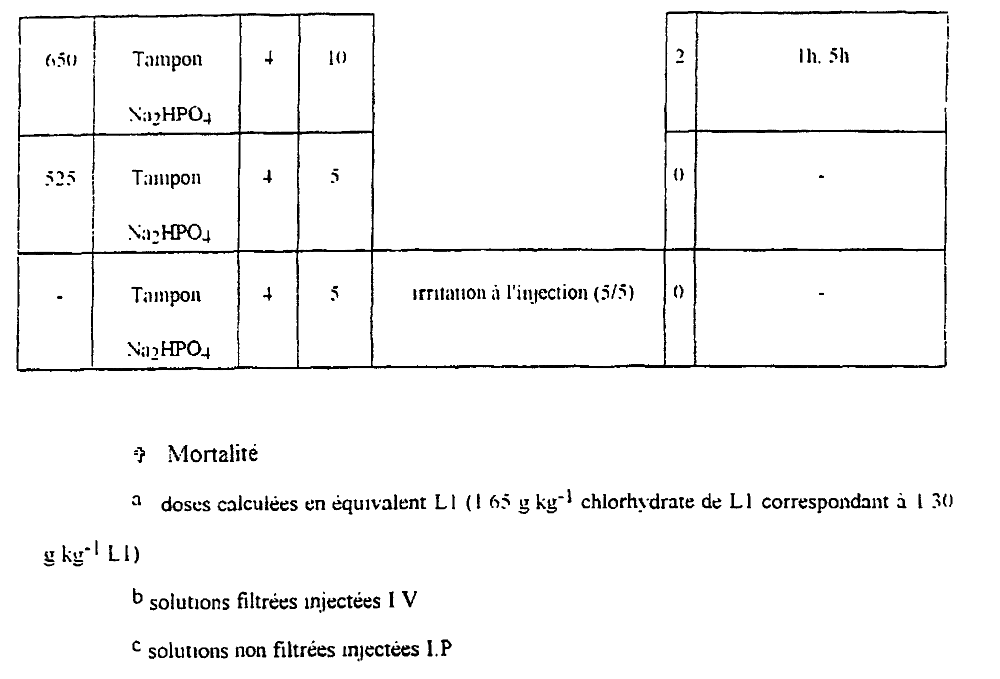

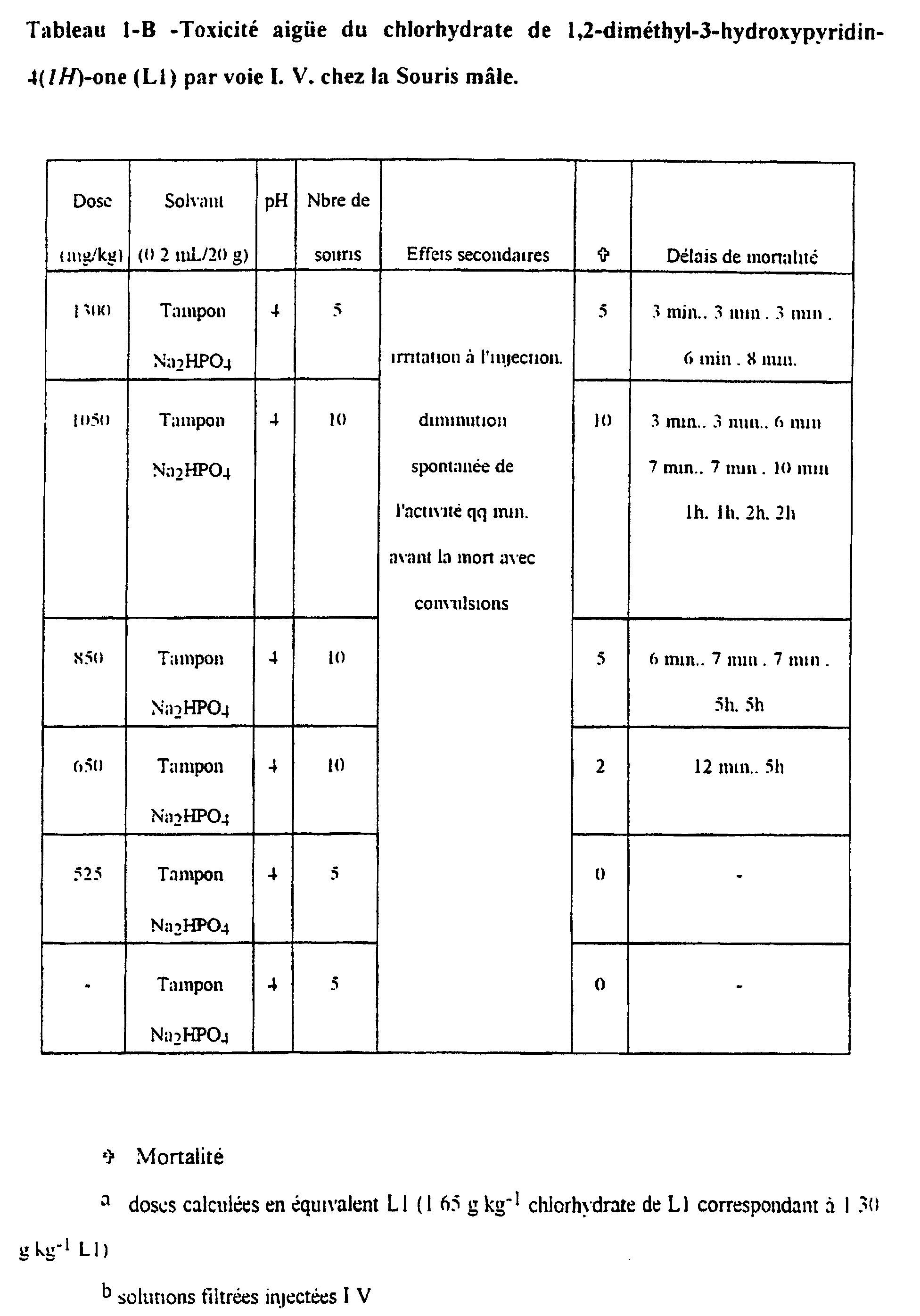

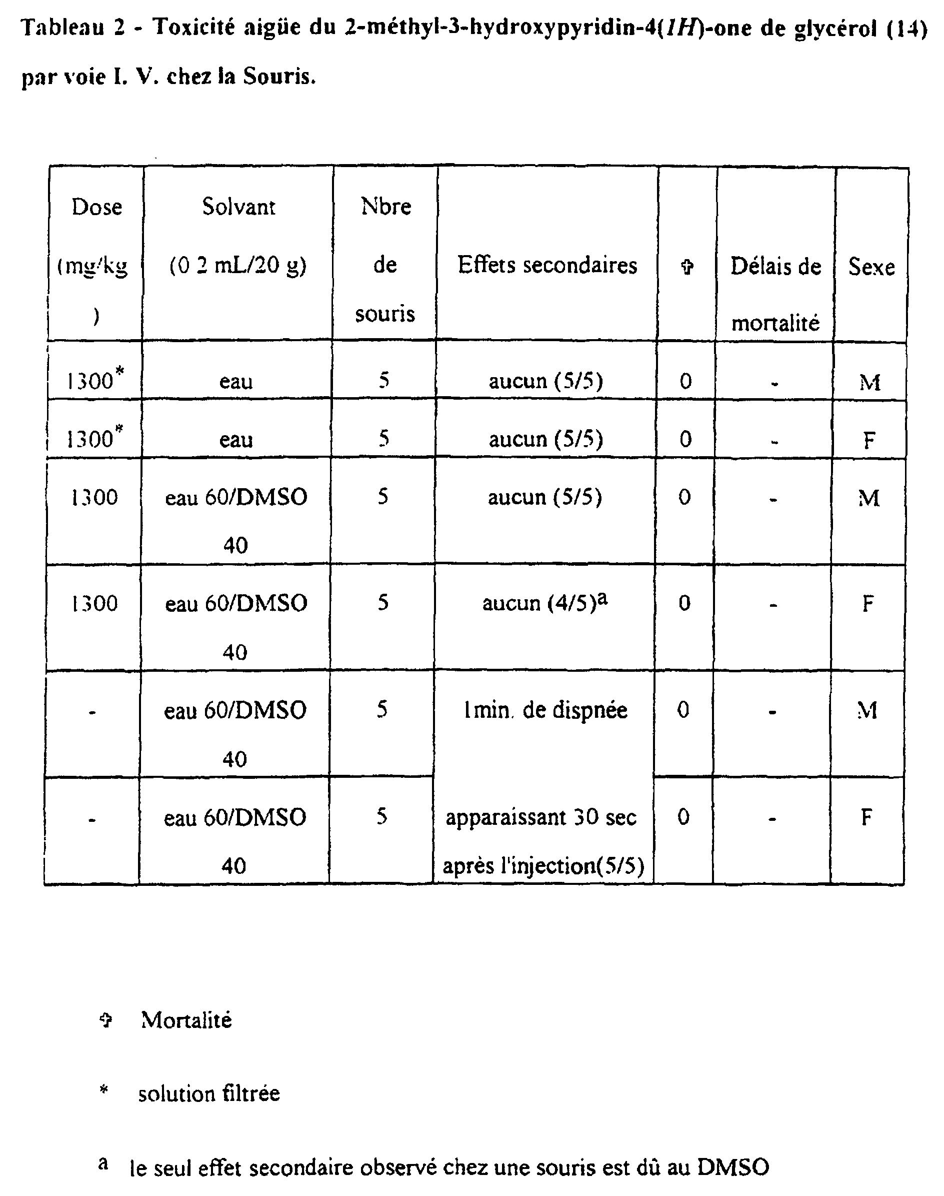

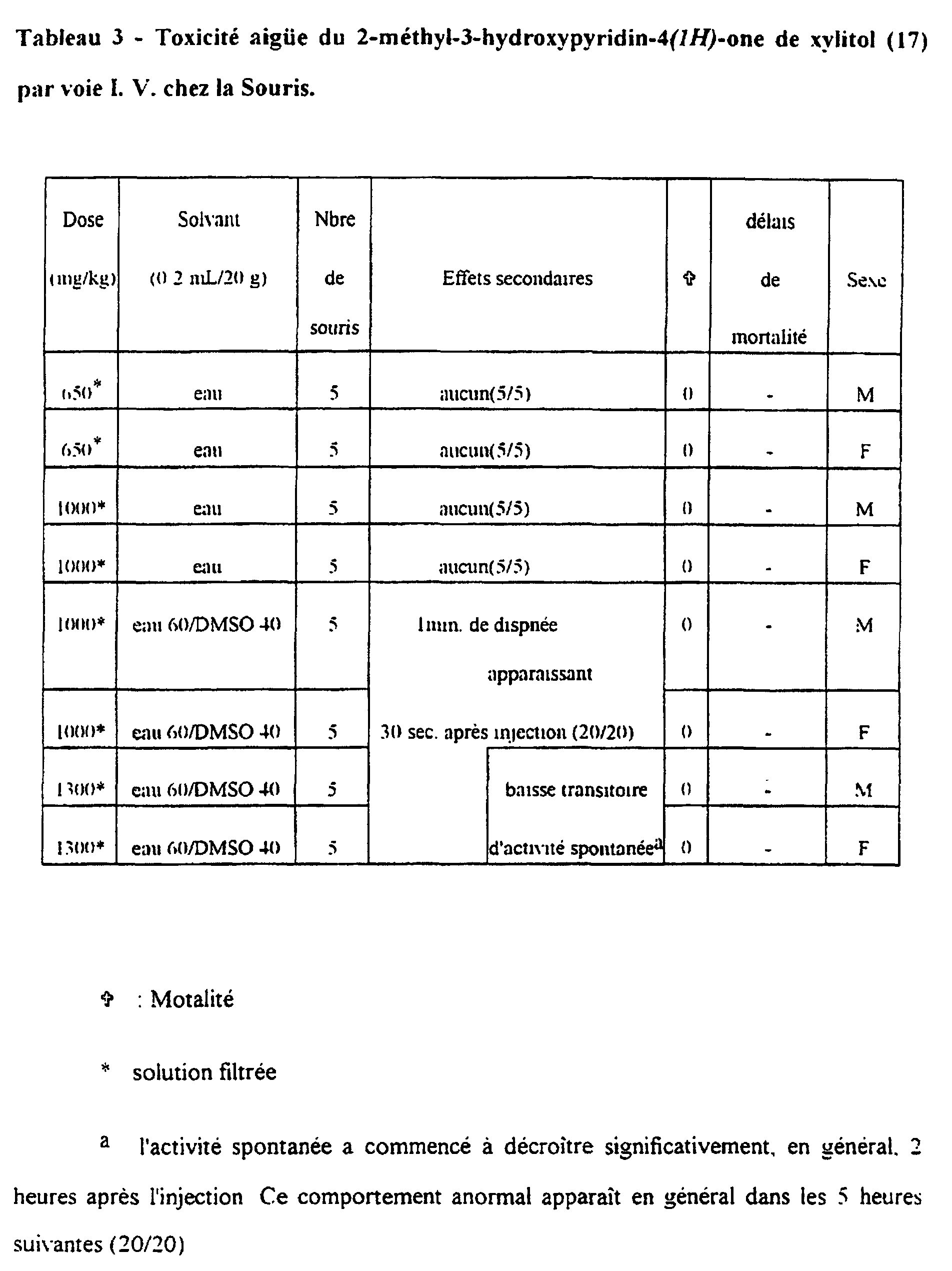

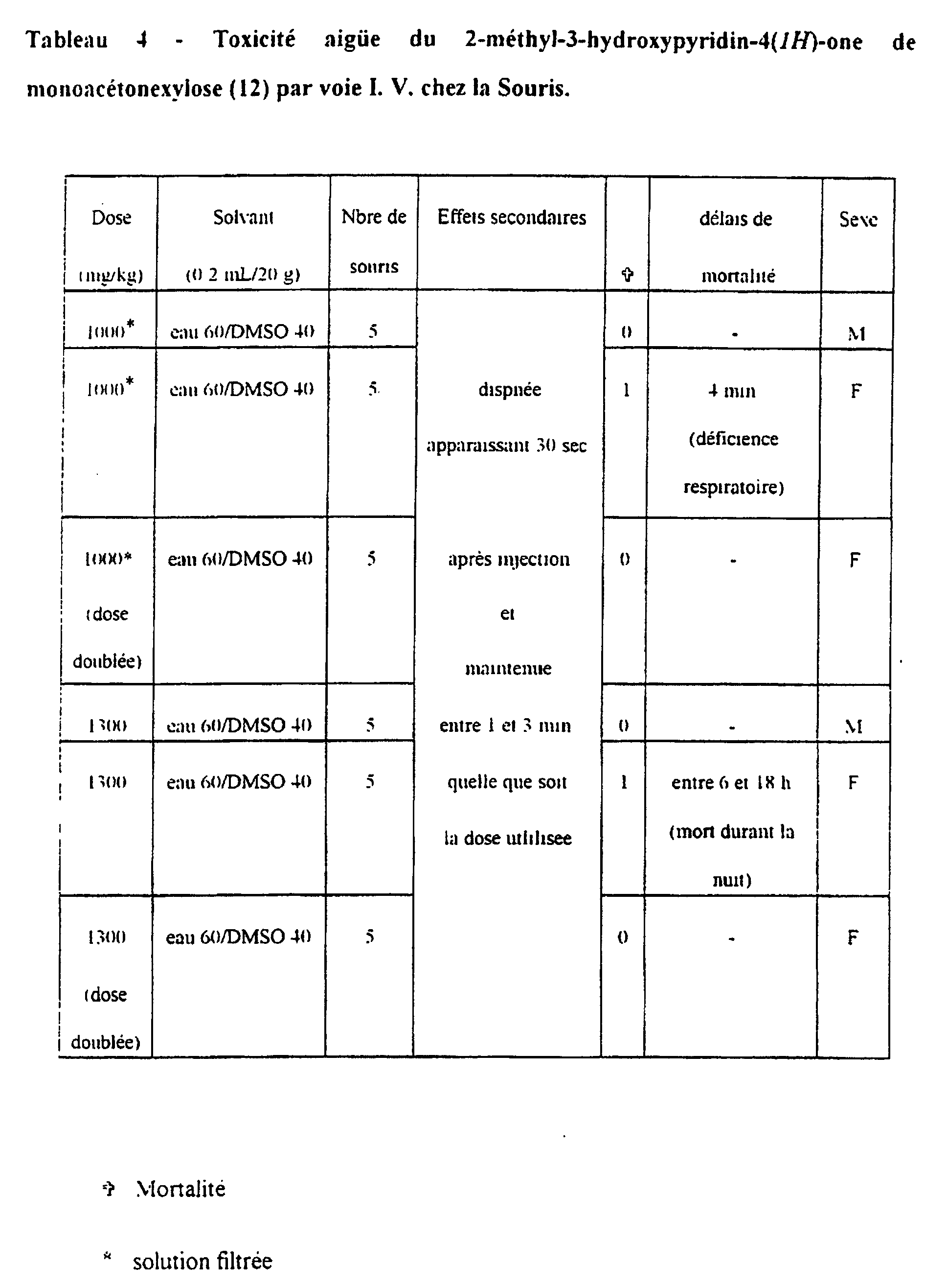

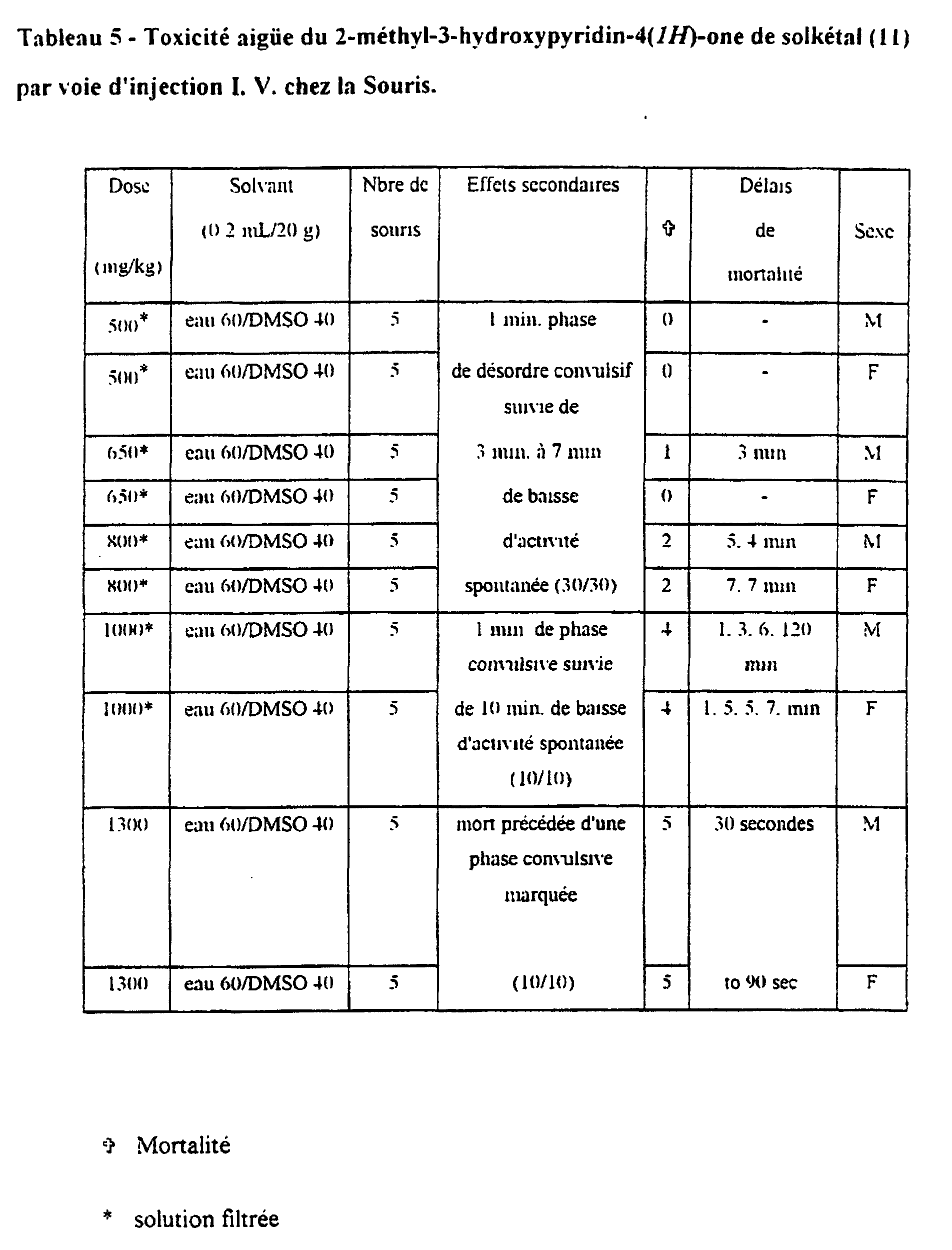

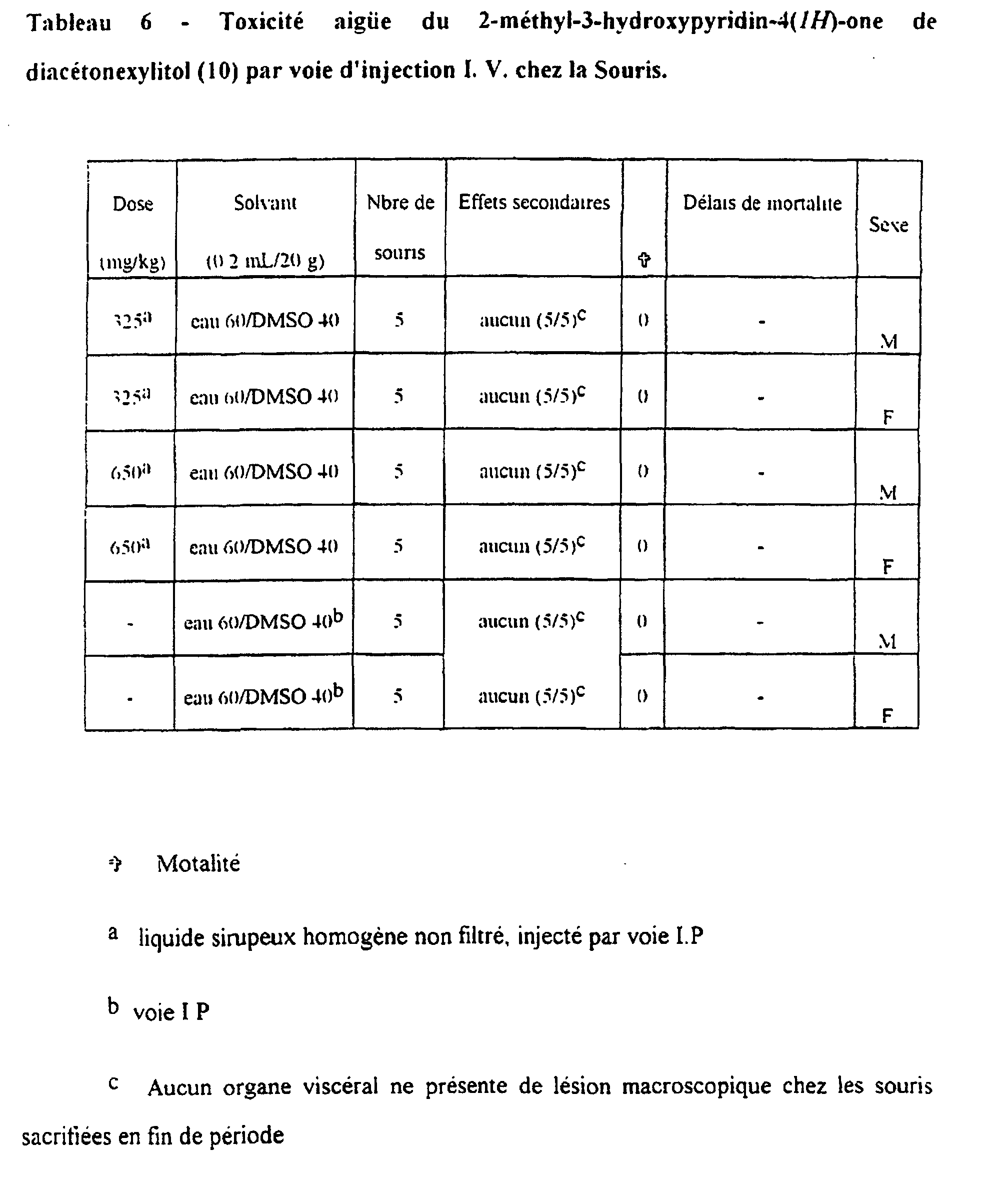

## Revendications

1. Procédé de synthèse régiospécifique de nouveaux dérivés de 3-hydroxypyridin-4(*1H*)-ones à partir d'aminoitols. de formule générale: 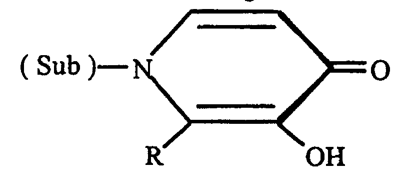 dans laquelle R représente un radical alkyle ou alkylène ou haloalkyle ou haloalkylène, les halogènes étant choisis de préférence parmi chlorure et fluorure, droit ou ramifié, ayant de 1 à 4 atomes de carbon et ayant des hétéroatomes ou non, et Sub représente un itol, cyclique ou non, protégé ou non, dont le squelette hydrocarboné est lié à l'atome d'azote de la pyridinone soit directement, soit par l'intermédiaire d'un bras d'espacement de type alkylène ou haloalkylène, les halogènes étant choisis de préférence parmi chlorure et fluorure, droit ou ramifié, ayant de 1 à 4 atomes de carbon et ayant des hétéroatomes ou non, **caractérisé en ce qu'**il comprend:
■ une première étape (étape a) de protection du groupement 3-hydroxy du dérivé pyranone;
■ une deuxième étape (étape b) de substitution de l'atome d'oxygène intracyclique de la pyranone par l'atome d'azote de la fonction amine de l'aminoitol, pour conduire à la 2-R-3-hydroxypyridin-4(*1H*)-one dérivée de l'itol, dans laquelle on fait réagir au moins 1 équivalent molaire de la pyranone protégée issue de l'étape (a) en présence d'une base forte sur 1 équivalent molaire de l'aminoitol portant le groupement amino soit directement sur l'itol soit par l'intermédiaire d'un bras d'espacement de type alkylène ou haloalkylène;
■ une troisième étape (étape c) de déprotection du groupement 3-OH du cycle pyridinone et éventuellement des groupements OH de l'itol, les groupements OH de l'itol peuvant également être déprotégés dans une quatrième étape (étape d).

2. Procédé selon la revendication 1 **caractérisé en ce que** le groupement Sub est choisi dans le groupe constitué par hexitol, pentitol, tétritol, glycérol notamment glucitol, galacitol, xylitol, érythritol, glycérol.

3. Procédé selon la revendication 1 et. 2 **caractérisé en ce que** les conditions opératoires de l'étape (a) visant à protéger le groupement 3-hydroxy du dérivé pyranone se caractérisent **en ce que** :
on fait réagir le dérivé 2-R-3-hydroxypyran-4-one avec au moins un équivalent molaire de dérivé P-X, dans lequel P représente un groupement alkyle, cyclique ou non, ramifié ou non, saturé ou non, ou encore un groupement phényle substitué ou non ou encore un groupement aryle substitué ou non, et X un groupement partant, l'étape (a) de protection étant conduite en présence d'au moins 1 équivalent molaire de base forte choisie par exemple parmi les bases hydroxylées ou encore parmi les alcanoates et les sels d'acides faibles, le cation pouvant être monovalent tel que Na⁺, K⁺, Li⁺, Rb⁺, Cs⁺ ou encore un cation polyvalent Mⁿ⁺ de type alcalino-terreux ou tout autre, le solvant de réaction pouvant être des mélanges hydroalcanoliques, l'alcanol étant choisi par exemple parmi le méthanol, l'éthanol, le propanol ou l'isobutanol ou encore un alcanol seul, ou encore un solvant aprotique polaire choisi par exemple parmi l'hexaméthylphosphorotriamide (HMPA), le diméthylformamide (DMF), le diméthoxyéthane (DME), le diméthylsulfoxyde (DMSO), l'acétone , ou encore un mélange de ces derniers, le solvant aprotique polaire pouvant être ou non associé à un solvant aprotique apolaire choisi par exemple parmi les solvants aromatiques, les hydrocarbures ou encore les éthéroxydes ou encore un mélange de ces solvants, l'étape (a) comprenant également les opérations suivantes :
• élimination du solvant et reprise du résidu par un solvant organique ;
• lavage de la phase organique successivement par une solution aqueuse de base, comme précédemment définie, puis par de l'eau ;
• évaporation des phases organiques précédemment obtenues, pour conduire au dérivé pyranone protégé en -3

4. Procédé selon la revendication 3 **caractérisé en ce que** le groupement P est choisi dans le groupe constitué par alkyle, benzyle et phényle.

5. Procédé selon la revendication 3 et 4, **caractérisé en ce que** X est choisi dans le groupe constitué par les halogénures et sulfonates notamment chlorure, bromure, iodure, tosylate, mésylate, brosylate, nosylate, triflate.

6. Procédé selon les revendications 1 et 2 **caractérisé en ce que** les conditions opératoires de l'étape (b) de substitution de l'atome d'oxygène intracyclique de la pyranone par l'atome d'azote de l'aminoitol se **caractérise en ce que** :
on fait réagir au moins 1 équivalent molaire de la pyranone protégée issue de l'étape (a), en présence d'une base forte comme défini à l'étape (a), selon la revendication 3, sur 1 équivalent molaire de l'aminoitol portant le groupement amino soit directement soit par l'intermédiaire d'un bras d'espacement, le solvant de l'étape (b)pouvant être des mélanges hydroalcanoliques, l'alcanol étant choisi par exemple parmi le méthanol, l'éthanol, le propanol ou l'isobutanol ou encore un alcanol seul, ou encore un solvant aprotique polaire choisi par exemple parmi l'hexaméthylphosphorotriamide (HMPA), le diméthylformamide (DMF), le diméthoxyéthane (DME), le diméthylsulfoxyde (DMSO), l'acétone , ou encore un mélange de ces derniers, le solvant aprotique polaire pouvant être ou non associé à un solvant aprotique apolaire choisi par exemple parmi les solvants aromatiques, les hydrocarbures ou encore les éthéroxydes ou encore un mélange de ces solvants, l'étape (b) comprenant de plus les opérations suivantes :
• neutralisation de la base par addition d'un acide minéral ou organique ;
• élimination du solvant organique ;
• reprise du résidu par un solvant organique et lavage de la phase organique par de l'eau ;
• évaporatipn de la phase organique ;
• purification du résidu par recristallisation ou chromatographie.

7. Procédé selon les revendications 1 à 6 **caractérisé en ce que** les conditions opératoires de l'étape (c) de déprotection du cycle pyridinone des composés issus de l'étape (b) se caractérisent **en ce que** :
la déprotection est réalisée pour P choisi parmi benzyle, phényle, allyle par hydrogénolyse en présence d'un catalyseur choisi parmi Pd/C, ou encore tout autre catalyseur d'hydrogénolyse choisi parmi les dérivés du platine, les dérivés du nickel, dans un alcanol ou encore un mélange alcanol-eau, l'alcanol étant choisi parmi le méthanol, l'éthanol, le propanol, l'isopropanol, ou encore un solvant organique associé ou non à l'eau et permettant la solubilisation du produit déprotégé, choisi parmi tétrahydrofurane, dioxane, l'étape (c) comprenant, de plus, les opérations suivantes :
• filtration du catalyseur ;
• élimination du solvant organique ;
• purification du résidu par exemple par recristallisation dans par exemple un mélange binaire de solvants organiques choisis par exemple parmi hexane, acétone.

8. Procédé selon les revendications 1 à 7 **caractérisé en ce que** l'étape (c) de déprotection du cycle pyridinone des composés issus de l'étape (b) peut être conduite, dans le cas de composés à reste de l'itol protégé par des groupements acidolabiles, dans les conditions de l'étape (c) selon la revendication 7 mais en présence d'un catalyseur acide de façon à opérer la déprotection des groupements protecteurs du reste de l'itol de façon "one-pot", le dérivé de l'itol de la 2-R-3-hydroxypyridin-4(*1H*)-one pouvant alors être obtenu soit sous forme de sel d'ammonium par lyophilisation du brut, soit sous forme d'amine après neutralisation du milieu réactionel par passage sur résine échangeuse d'ions de nature basique choisie parmi Dowex® Amberlyst® ou Amberlite®.

9. Procédé selon les revendications 1 à 6 **caractérisé en ce que** l'étape (c) de déprotection du cycle pyridinone des composés issus de l'étape (b) peut être conduite lorsque P est un groupement allyle, par hydrolyse acidocatalysée, après isomérisation basocatalysée du groupement allyle en groupement propényle, dans les solvants ou mélange de solvants décrits à la revendication 7.

10. Procédé selon les revendications 1-7 et 9 **caractérisé en ce que** les composés issus de l'étape (c) dont le reste de l'itol possède des groupement hydroxyles protégés peuvent être soumis à une étape (d) de déprotection selon les conditions classiques choisis en fonction de la nature de ces mêmes groupements protecteurs.

11. Nouvelles 2-R-3-hydroxypyridin-4(*1H*)-ones, dérivées d'itols, répondant à la formule suivante: 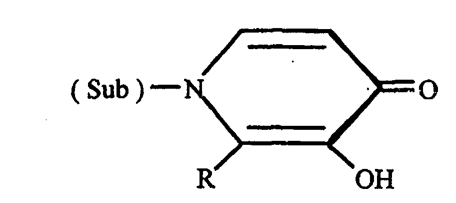 dans laquelle R représente un radical alkyle ou alkylène ou haloalkyle ou haloalkylène, ayant de 1 à 4 atomes de carbone et ayant des hétéroatomes ou non, et Sub représente un itol, choisi parmi hexitol, pentitol, tétritol, glycérol notamment glucitol, galacitol, xylitol, érythritol, glycérol, dont le squelette hydrocarboné est lié à l'atome d'azote de la pyridinone soit directement, soit par l'intermédiaire d'un bras d'espacement de type alkylène ou haloalkylène, droit ou ramifié, ayant de 1 à 4 atomes de carbone, des hétéroatomes ou non.

12. Les 1-(2',3' : 4',5'-di-( )-isopropylidène-xylityl)-2-méthyl-3-benzyloxypyridin-4(*1H*)-one et 1-(2',3' : 4',5'-di- ( ) -isopropylidène-xylityl)-2-méthyl-3-hydroxypyridin-4(*1H*)-one.

13. Les 1-(2',4' : 3',5'-di- ( ) -méthylène-xylityl)-2-méthyl-3-benzyloxypyridin-4(*1H*)-one et 1-(2',4' : 3',5'-di- ( ) -méthylène-xylityl)-2-méthyl-3-hydroxypyridin-4(*1H*)-one.

14. Les 1-(2',3'- ( ) -isopropylidène-glycéryl)-2-méthyl-3-benzyloxypyridin-4(*1H*)-one et 1-(2',3'- ( ) -isopropylidène-glycéryl)-2-méthyl-3-hydroxypyridin-4(*1H*)-one.

15. Le 1-glycéryl-2-méthyl-3-benzyloxypyridin-4(*1H*)-one.

16. Le 1-glycéryl-2-méthyl-3-hydroxypyridin-4(*1H*)-one.

17. Le 1-xylityl-2-méthyl-3-hydroxypyridin-4(*1H*)-one.

18. Chélatants des métaux comprenant les 2-R-3-hydroxypyridin-4(*1H*)-ones , dérivés d'itols selon l'une quelconque des revendications 11 à 17.

19. Médicaments pour le traitement des surcharges toxiques en métaux, notamment Fe^{III} , comportant une quantité thérapeutiquement efficace de l'un au moins des composés, selon l'une des revendication 11 à 17, dans un véhicule administrable à l'Homme.

20. Utilisation d'un composé selon l'une des revendications 11 à 17, pour la préparation d'un médicaments pour le traitement des surcharges toxiques en métaux, y compris Fe^{III}, et notamment pour le traitement des hémoglobinopathies, la β-thalassémie, la drépanocytose.

## Claims

1. Process for the regiospecific synthesis of new 3-hydroxypyridin-4(1H)-one derivatives of aminoitols, of general formula: 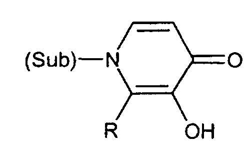 wherein R denotes an alkyl or alkylene or haloalkyl or haloalkylene radical, the halogens preferably being selected from among chlorine and fluorine, straight-chain or branched, having 1 to 4 carbon atoms and with or without heteroatoms, and Sub denotes a cyclic or non-cyclic protected or unprotected itol the hydrocarbon skeleton of which is bonded to the nitrogen atom of the pyridone either directly or via a spacer arm of the alkylene or haloalkylene type, the halogens preferably being selected from among chlorine and fluorine, straight-chained or branched, having 1 to 4 carbon atoms and with or without heteroatoms, **characterised in that** it comprises:
a first step (step a) of protecting the 3-hydroxy group of the pyranone derivative;
a second step (step b) of substituting the intracyclic oxygen atom of the pyranone with the nitrogen atom of the amine function of the aminoitol, to produce the 2-R-3-hydroxypyridin-4(1H)-one derived from the itol, wherein at least one molar equivalent of the protected pyranone from step (a) is reacted in the presence of a strong base with one molar equivalent of the aminoitol carrying the amino group either directly on the itol or via a spacer arm of the alkylene or haloalkylene type;
a third step (step c) of deprotecting the 3-OH group of the pyridone ring and optionally the OH groups of the itol, while the OH groups of the itol may also be deprotected in a fourth step (step d).

2. Process according to claim 1, **characterised in that** the Sub group is selected from the group consisting of hexitol, pentitol, tetritol, glycerol particularly glucitol, galacitol, xylitol, erythritol and glycerol.

3. Process according to claim 1 and 2, **characterised in that** the operating conditions of step (a) for protecting the 3-hydroxy group of the pyranone derivative are **characterised in that**:
the 2-R-3-hydroxypyran-4-one derivative is reacted with at least one molar equivalent of the derivative P-X, wherein P denotes a cyclic or non-cyclic, straight-chain or branched, saturated or unsaturated alkyl group, or a substituted or unsubstituted phenyl group or a substituted or unsubstituted aryl group, and X denotes a leaving group, the protecting step (a) being carried out in the presence of at least one molar equivalent of strong base selected, for example, from among the hydroxylated bases or from the alkanoates and weak acid salts, while the cation may be monovalent such as Na⁺, K⁺, Li⁺, Rb⁺, Cs⁺ or a polyvalent cation Mⁿ⁺ of the alkaline earth type or any other type, the solvent for the reaction optionally being a hydroalkanolic mixture, the alkanol being selected, for example, from among methanol, ethanol, propanol or isobutanol, or optionally being an alkanol alone, or a polar aprotic solvent selected, for example, from among hexamethylphosphorotriamide (HMPA), dimethylformamide (DMF), dimethoxyethane (DME), dimethylsulphoxide (DMSO), acetone or a mixture of the latter, while the polar aprotic solvent may or may not be combined with an aprotic non-polar solvent selected for example from among the aromatic solvents, the hydrocarbons or the ether oxides or a mixture of these solvents, step (a) also including the following operations:
eliminating the solvent and taking up the residue in an organic solvent;
washing the organic phase successively with an aqueous solution of base as hereinbefore defined and then with water;
evaporating the organic phases obtained previously to obtain the pyranone derivative protected in the 3-position.

4. Process according to claim 3, **characterised in that** the group P is selected from among alkyl, benzyl and phenyl.

5. Process according to claim 3 or 4, **characterised in that** X is selected from among the halides and sulphonates, particularly chloride, bromide, iodide, tosylate, mesylate, brosylate, nosylate and triflate.

6. Process according to claims 1 and 2, **characterised in that** the operating conditions of step (b) for substituting the intracyclic oxygen atom of the pyranone with the nitrogen atom of the aminoitol are **characterised in that**:
at least one molar equivalent of the protected pyranone from step (a) is reacted in the presence of a strong base as defined in step (a), according to claim 3, with one molar equivalent of the aminoitol carrying the amino group either directly or via a spacer arm, the solvent of step (b) optionally being a hydroalkanolic mixture, the alkanol being selected, for example, from among methanol, ethanol, propanol or isobutanol, or optionally being an alkanol alone, or a polar aprotic solvent selected, for example, from among hexamethylphosphorotriamide (HMPA), dimethylformamide (DMF), dimethoxyethane (DME), dimethylsulphoxide (DMSO), acetone, or a mixture of the latter, while the polar aprotic solvent may or may not be combined with a non-polar aprotic solvent selected, for example, from among the aromatic solvents, the hydrocarbons or the ether oxides or a mixture of these solvents, step (b) additionally comprising the following operations:
neutralising the base by the addition of an inorganic or organic acid;
eliminating the organic solvent;
taking up the residue in an organic solvent and
washing the organic phase with water;
evaporating the organic phase;
purifying the residue by recrystallisation or chromatography.

7. Process according to claims 1 to 6, **characterised in that** the operating conditions of step (c) of deprotecting the pyridinone ring of the compounds obtained in step (b) are **characterised in that**:
the deprotection is carried out for P selected from among benzyl, phenyl or allyl by hydogenolysis in the presence of a catalyst selected from Pd/C, or any other hydrogenolysis catalyst selected from among the platinum derivatives, the nickel derivatives, in an alkanol or a mixture of alkanol and water, the alkanol being selected from among methanol, ethanol, propanol, isopropanol, or an organic solvent which may or may not be combined with water and allowing solubilisation of the deprotected product, selected from among tetrahydrofuran and dioxan, step (c) further comprising the following operations:
filtration of the catalyst;
elimination of the organic solvent;
purification of the residue, for example by recrystallisation, for example from a binary mixture of organic solvents selected, for example, from hexane and acetone.

8. Process according to claims 1 to 7, **characterised in that** step (c) of deprotecting the pyridinone ring of the compounds obtained in step (b) may be carried out, in the case of compounds with an itol residue protected by groups which are unstable in acid, under the conditions of step (c) according to claim 7, but in the presence of an acid catalyst so as to carry out the deprotection of the protecting groups of the itol residue in a one-pot process, the itol derivative of 2-R-3-hydroxypyridin-4(1H)-one then optionally being obtained either in the form of an ammonium salt by lyophilisation of the crude product or in the form of an amine after neutralisation of the reaction medium by passing it over an ion exchange resin of a basic type selected from among Dowex®, Amberlyst® or Amberlite®.

9. Process according to claims 1 to 6, **characterised in that** step (c) of deprotecting the pyridinone ring of the compounds obtained in step (b) may be carried out by acid-catalysed hydrolysis when P is an allyl group, after base-catalysed isomerisation of the allyl group into a propenyl group, in the solvents or mixture of solvents described in claim 7.

10. Process according to claims 1-7 and 9, **characterised in that** the compounds obtained in step (c), wherein the itol residue has protected hydroxyl groups, may be subjected to a step (d) of deprotection under conventional conditions selected according to the nature of these same protecting groups.

11. New 2-R-3-hydroxypyridin-4(1H)-ones derived from itols, corresponding to the following formula: 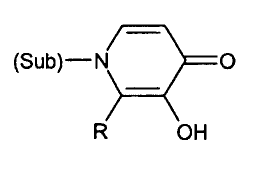 wherein R denotes an alkyl or alkylene or haloalkyl or haloalkylene radical having 1 to 4 carbon atoms and with or without heteroatoms and Sub denotes an itol selected from among hexitol, pentitol, tetritol, glycerol particularly glucitol, galacitol, xylitol, erythritol and glycerol, wherein the hydrocarbon skeleton is bonded to the nitrogen atom of the pyridinone either directly or via a spacer arm of the alkylene or haloalkylene type, straight-chained or branched, having 1 to 4 carbon atoms, with or without heteroatoms.

12. 1-(2,3':4',5'-di-()-isopropylidene-xylityl)-2-methyl-3-benxyloxypyridin-4(1H)-one and 1-(2',3':4',5'-di-() -isopropylidene-xylityl) -2-methyl-3-hydroxypyridin-4(1H)-one.

13. 1-(2',4':3',5'-di-()-methylene-xylityl)-2-methyl-3-benxyloxypyridin-4(1H)-one and 1-(2',4':3',5'-di-()-methylene-xylityl)-2-methyl-3-hydroxypyridin-4(1H)-one.

14. 1-(2',3-()-isopropylidene-glyceryl)-2-methyl-3-benzyloxypyridin-4(1H)-one and 1-(2',3'-()-isopropylidene-glyceryl)-2-methyl-3-hydroxypyridin-4(1H)-one.

15. 1-glyceryl-2-methyl-3-benxyloxypyridin-4(1H)-one.

16. 1-glyceryl-2-methyl-3-hydroxypyridin-4(1H)-one.

17. 1-xylityl-2-methyl-3-hydroxypyridin-4(1H)-one.

18. Metal chelating agents containing the 2-R-3-hydroxypyridin-4(1H)-ones derived from itols according to any one of claims 11 to 17.

19. Medicaments for treating toxic overloads of metals, particularly Fe(III), containing a therapeutically effective amount of at least one of the compounds according to one of claims 11-17 in a carrier which can be administered to humans.

20. Use of a compound according to one of claims 11-17 for preparing a medicament for treating toxic overloads of metals, including Fe(III), and particularly for treating haemoglobinopathies, β-thalassaemia and sickle cell anaemia.

## Patentansprüche

1. Verfahren zur regioselektiven Synthese von neuen Derivaten der 3-Hydroxypyridin-4(*1H*)-one ausgehend von Aminozuckeralkoholen der allgemeinen Formel: 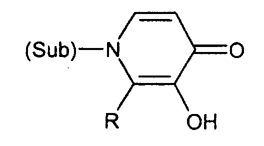 in der R ein gerades oder verzweigtes Alkyl- oder Alken- oder Halogenalkyl- oder Haloalkenradikal mit 1 bis 4 Kohlenstoffatomen und mit oder ohne Heteroatomen darstellt, wobei die Halogenen vorzugsweise unter Chlor und Fluor ausgewählt werden, und *Sub* einen cyclischen oder nicht cyclischen, geschützten oder ungeschützten Zuckeralkohol darstellt, dessen Kohlenwasserstoffgerüst mit einem Stickstoffatom verknüpft ist, entweder direkt oder indirekt über einen Abstandshalter vom Alken- oder Halogenalkentyp, gerade oder verzweigt mit 1 bis 4 Kohlenstoffatomen und mit oder ohne Heteroatome, wobei das Halogen vorzugsweise unter Fluor und Chlor ausgewählt wird, **dadurch gekennzeichnet, dass** es umfasst
- einen ersten Schritt (Schritt a) der Schutzgruppeneinführung an der 3-Hydroxygruppe des Pyranonderivates;
- einen zweiten Schritt (Schritt b) der Substitution des intracyclischen Sauerstoffatoms des Pyranons durch eine Stickstoffatom mit Aminfunktion im Aminozuckeralkohol, um zu dem 2-R-3-Hydroxypyridin-4(*1H*)-onderivat des Zuckeralkohols zu führen, in dem man mindestens 1 Moläquivalent des geschützten Pyranons aus Schritt (a) in Gegenwart einer starken Base mit 1 Moläquivalent des Aminozuckeralkohols, bei dem sich die Aminogruppe direkt am Zuckeralkohol oder an einem Abstandshalter vom Alken- oder Halogenalkentyp befindet;
- einen dritten Schritt (Schritt c) der Schutzgruppenabspaltung an der 3-OH-Gruppe des Pyridinonringes und eventueller OH-Gruppen im Zuckeralkohol, wobei Schutzgruppen der OH-Gruppen des Zuckeralkohols auch in einem vierten Schritt (Schritt d) abgespalten werden können.

2. Verfahren nach Anspruch 1, **dadurch gekennzeichnet, dass** die Sub-Gruppe ausgewählt wird aus der Reihe bestehend aus Hexit, Pentit, Tetrit, Glycerin und vor allem Glucit, Galacit, Xylit, Erythrit, Glycerin.

3. Verfahren nach Anspruch 1 und 2, **dadurch gekennzeichnet, dass** die Arbeitsbedingungen von Schritt (a), der die 3-Hydroxygruppe des Pyranonderivats schützen soll, **dadurch gekennzeichnet sind, dass**
man das 2-R-3-Hydroxypyran-4-onderivat mit mindestens einem Moläquivalent eines P-X-Derivates reagieren lässt, wobei P eine cyclische oder nicht cyclische, verzweigte oder unverzweigte, gesättigte oder ungesättigte Alkylgruppe oder auch eine substituierte oder unsubstituierte Phenylgruppe oder auch eine substituierte oder unsubstituierte Arylgruppe und X eine Abgangsgruppe darstellt, wobei der Schritt (a) der Schutzgruppeneinführung in Gegenwart von 1 Moläquivalent einer starken Base ausgerührt wird, welche zum Beispiel unter den Hydroxybasen oder auch unter den Alkanolaten und den Salzen schwacher Säuren ausgewählt wird, wobei das Kation monovalent wie Na⁺, K⁺, Li⁺, Rb⁺, Cs⁺ oder auch ein polyvalentes Kation Mⁿ⁺ vom Typ der Erdalkalimetalle oder jedes andere sein kann, und das Lösungsmittel der Reaktion ein Gemisch aus Wasser und Alkanolen sein kann, wobei der Alkohol zum Beispiel ausgewählt wird unter Methanol, Ethanol, Propanol, Isobutanol, oder auch ein Alkanol allein sein kann oder ein aprotisches, polares Lösungsmittel, welches zum Beispiel ausgewählt wird unter Hexamethylphosphortriamid (HMPA), Dimethylformamid (DMF) Dimethoxyethan (DME), Dimethylsulfoxid (DMSO) oder Aceton, oder ein Gemisch der letzteren, wobei das aprotische, polare Lösungsmittel mit einem aprotischen, unpolaren Lösungsmittel verbunden werden kann oder nicht, welches zum Beispiel ausgewählt werden kann aus aromatischen Lösungsmittel, Kohlenwasserstoffen oder auch Ether, oder auch ein Gemisch dieser Lösungsmittel, wobei Schritt (a) auch folgende Arbeitsschritte umfasst:
- Entfernen des Lösungsmittels und Aufnahme des Rückstandes durch ein organisches Lösungsmittel
- Waschen der organischen Phasen nacheinander mit einer basischen wässrigen Lösung, definiert wie vorher beschrieben, dann mit Wasser;
- Eindampfen der vorher erhaltenen organischen Phasen, um das an Position 3 geschützte Pyranonderivat zu erhalten.

4. Verfahren nach Anspruch 3, **dadurch gekennzeichnet, dass** die P-Gruppe ausgewählt wird aus der Gruppe bestehend aus Alkyl-, Benzyl- und Phenyl-.

5. Verfahren nach Anspruch 3 und 4 **dadurch gekennzeichnet, dass** X aus der Gruppe ausgewählt wird bestehend aus Halogeniden und Sulfonaten, vor allem Chlorid, Bromid, Iodid, Tosylat, Mesylat, p-Brombenzolsulfonat (Brosylat), p-Nitrobenzolsulfonat (Nosylat), Triflat.

6. Verfahren nach Anspruch 1 und 2, **dadurch gekennzeichnet, dass** die Arbeitsbedingungen bei Schritt (b), der Substitution des intracyclischen Sauerstoffatoms des Pyranons durch ein Stickstoffatom des Aminozuckeralkohols, **dadurch gekennzeichnet sind, dass**
man mindestens 1 Moläquivalent des geschützten Pyranons aus Schritt (a) reagieren lässt in Gegenwart einer starken Base wie sie in Schritt (a) nach Anspruch 3 definiert wurde, mit 1 Moläquivalent des Aminozuckeralkohols, welcher die Aminogruppe direkt oder an einem Abstandshalter trägt, wobei das Lösungsmittel des Schritt (b) ein Wasser/Alkanol-Gemisch sein kann, wobei das Alkanol zum Beispiel ausgewählt wird aus Methanol, Ethanol, Propanol oder Isobutanol, oder auch ein Alkanol allein sein kann, oder auch ein aprotisches, polares Lösungsmittel, welches zum Beispiel ausgewählt wird unter Hexamethylphosphortriamid (HMPA), Dimethylformamid (DMF) Dimethoxyethan (DME), Dimethylsulfoxid (DMSO) oder Aceton, oder ein Gemisch der letzteren, wobei das aprotische ,polare Lösungsmittel mit einem aprotischen, unpolaren Lösungsmittel verbunden werden kann oder nicht, welches zum Beispiel ausgewählt werden kann aus aromatischen Lösungsmittel, Kohlenwasserstoffen oder auch Ether, oder auch ein Gemisch dieser Lösungsmittel, wobei Schritt (b) weiterhin folgende Arbeitsschritte umfasst:
- Neutralisation der Base durch Hinzufügen einer mineralischen oder organischen Säure;
- Entfernen des organischen Lösungsmittels;
- Aufnahme des Rückstandes durch ein organisches Lösungsmittel und Waschen der organischen Phase mit Wasser;
- Eindampfen der organischen Phase;
- Reinigung des Rückstandes durch Rekristallisation oder Chromatographie.

7. Verfahren nach den Ansprüchen 1 bis 6, **dadurch gekennzeichnet, dass** die Arbeitsbedingungen des Schrittes (c), der Schutzgruppenabspaltung des Pydridinonringes der aus Schritt (b) erhaltenen Verbindung, **dadurch gekennzeichnet ist, dass** die Abspaltung der Schutzgruppe für P, ausgewählt aus Benzyl-, Phenyl- und Allyl-, durch Hydrogenolyse in Gegenwart eines Katalysators ausgewählt aus Pd/C oder auch allen anderen Katalysatoren für die Hydrogenolyse ausgewählt unter Platinderivaten oder Nickelderivaten, in einem Alkanol oder auch in einem Alkanol/Wasser-Gemisch, wobei das Alkanol ausgewählt wird unter Methanol, Ethanol, Propanol, Isopropanol oder auch einem organischen Lösungsmittel, das mit Wasser verbunden ist oder nicht und ein Lösen des entschützten Produktes ermöglicht, ausgewählt aus Tetrahydrofuran oder Dioxan, wobei Schritt (c) weiterhin folgende Arbeitsschritte umfasst:
- Filtration des Katalysators;
- Entfernen des organischen Lösungsmittels
- Reinigung des Rückstandes zum Beispiel durch Rekristallisation in zum Beispiel einem binären Gemisch aus organischen Lösungsmitteln zum Beispiel ausgesucht unter Hexan und Aceton.

8. Verfahren nach den Ansprüchen 1 bis 7, **dadurch gekennzeichnet, dass** der Schritt (c) der Schutzgruppenabspaltung am Pyridinonring der Verbindungen aus Schritt (b) im Fall von Verbindungen mit einem Zuckeralkoholrest, der durch säureempfindliche Gruppen geschützt ist, unter den Bedingungen wie Schritt (c) nach Anspruch 7 ausgeführt werden kann, aber in Gegenwart eines Säurekatalysators, um die Abspaltung der Schutzgruppe vom Zuckeralkoholrest sozusagen in einer "Ein-Topf-Weise" auszuführen, wobei das Zuckeralkoholderivat des 2-R-3-Hydroxypyridin-4(1H)-ons dann erhalten werden kann entweder in Form eines Ammoniumsalzes durch Lyophilisierung des Rohprodukts oder in Form eines Amins nach der Neutralisation des Reaktionsmilieus, in dem man es durch einen basischen Ionentauscherharz laufen lässt, ausgewählt unter Dowex®, Amberlyst® oder Amberlite®.

9. Verfahren nach den Ansprüchen 1 bis 6, **dadurch gekennzeichnet, dass** die Schutzgruppenabspaltung am Pyridinonring der aus Schritt (b) erhaltenen Verbindungen, wenn P eine Allylgruppe ist, durch eine säurekatalysierte Hydrogenolyse erfolgen kann, nach einer basenkatalysierten Isomerisierung der Allylgruppe in eine Propenylgruppe in Lösungsmitteln oder Lösungsmittelgemischen wie in Anspruch 7 beschrieben.

10. Verfahren nach den Ansprüchen 1 - 7 und 9, **dadurch gekennzeichnet, dass** die aus Schritt (c) erhaltenen Verbindungen, deren Zuckeralkoholrest geschützte Hydroxylgruppen besitzt, einem Schritt (d) unterzogen werden können, einer Schutzgruppenabspaltung unter den klassischen Bedingungen in Abhängigkeit von der Natur dieser Schutzgruppen.

11. Neue 2-R-3-Hydroxylpyridin-4(*1H*)-one, Zuckeralkoholderivate, die der folgenden Formel entsprechen: 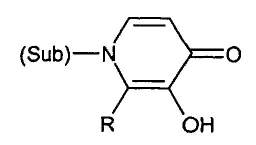 in der R ein Alkyl- oder Alken- oder Halogenalkyl- oder Halogenalkenradikal mit 1 bis 4 Kohlenstoffatomen und mit oder ohne Heteroatomen darstellt und *Sub* Zuckeralkohol darstellt, ausgewählt unter Hexit, Pentit, Tetrit, Glycerin, vor allem Glucit, Galacit, Xylit, Erythrit und Glycerin, dessen Kohlenwasserstoffgerüst mit einem Stickstoffatom verknüpft ist, entweder direkt oder indirekt über einen Abstandshalter vom Alken- oder Halogenalkentyp, welcher gerade oder verzweigt mit 1 bis 4 Kohlenstoffatomen und mit oder ohne Heteroatomen vorliegt.

12. Das 1-(2',3':4',5'-di-()-isopropylidenxylityl)-2-methyl-3-benzyloxypyridin-4(*1H*)-on und das 1-(2',3':4',5'-di-()-isopropylidenxylityl)-2-methyl-3-hydroxypyridin-4(*1H*)-on.

13. Das 1-(2',4':3',5'-di-()-methylenxylityl)-2-methyl-3-benzyloxypyridin-4(*1H*)-on und das 1-(2',4':3',5'-di-()-methylenxylityl)-2-methyl-3-hydroxypyridin-4(*1H*)-on.

14. Das 1-(2',3'-()-isopropylidenglyceryl)-2-methyl-3-benzyloxypyridin-4(*1H*)-on und das 1-(2',3'-()-isopropylidenglyceryl)-2-methyl-3-hydroxypyridin-4(*1H*)-on.

15. Das 1-Glyceryl-2-methyl-3-benzyloxypyridin-4(1H)-on.

16. Das 1-Glyceryl-2-methyl-3-hydroxypyridin-4(1H)-on.

17. Das 1-Xylityl-2-methyl-3-hydroxypyridin-4(1H)-on.

18. Metallchelatbildner, welche die 2-R-3-Hydroxypyridin-4(1H)-one, die Zuckeralkohol-derivate, nach einem der Ansprüche 11 bis 17 enthalten.

19. Medikamente für die Behandlung von toxischen Metallüberbelastungen, vor allem von Fe^{III}, welche eine effiziente therapeutische Menge von mindestens einer der Verbindungen nach Anspruch 11 bis 17 in einem Träger enthalten, der dem Menschen verabreicht werden kann.

20. Verwendung einer der Verbindungen nach einem der Ansprüche 11 bis 17 zur Herstellung von Medikamenten zur Behandlung von toxischen Metallüberbelastungen einschließlich Fe^{III} und vor allem zur Behandlung von Hämoglobinopathien, der β-Thalassämie und der Drepanozytose.
